# EUROPEAN PATENT APPLICATION

(11) **EP 4 714 342 A1**
(43) Date of publication of application: **25.03.2026**
(21) Application number: 24939216.8
(22) Date of filing: 24.05.2024
(51) Int. Cl.: A61B 5/0245, A61B 5/256

(54) **PHYSIOLOGICAL SIGNAL MONITORING DEVICE**

(71) Applicant: Ascend Technology Limited, Hong Kong 999077 (HK); Donghua University, Shanghai 200051 (CN)
(72) Inventor: ZHOU, Xin, Shenzhen, Guangdong 518108 (CN); LIU, Yujing, Shanghai 200051 (CN); WANG, Yongrong, Shanghai 200051 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2024/095259
(87) International publication number: WO 2025/241187

(57) **Abstract**

The embodiments of the present disclosure relate to a device for monitoring a physiological signal. The device includes a strap, two electrode elements, and a waterproof layer. The strap is configured to be worn on a body of a user. The two electrode elements are arranged at intervals in a length direction of the strap, each of the two electrode elements including an electrode configured to contact the body of the user to collect a physiological signal. The two electrode elements are both connected to the strap through the waterproof layer. The strap includes at least an elastic yarn and an insulating yarn. The waterproof layer includes a waterproof insulating yarn, and the strap and the waterproof layer are formed by integrated weaving.

## Description

### TECHNICAL FIELD

The present disclosure relates to the field of signal monitoring, and in particular, to a device for monitoring a physiological signal.

### BACKGROUND

As a device for monitoring a physiological signal, a heart rate strap may be provided with two electrocardiogram (ECG) electrodes, and an electrocardiogram signal may be determined based on a potential difference between positions of the two ECG electrodes. However, when a user wearing the heart rate strap performs exercise, sweat of the user may wet a substrate of the heart rate strap, thereby causing the substrate of the heart rate strap to become conductive. As a result, abnormal conduction may occur between the two ECG electrodes, which may lead to a reduction in an amplitude of the electrocardiogram signal collected by the heart rate strap and a decrease in a signal-to-noise ratio.

Accordingly, there is a need to provide a device for monitoring a physiological signal, which has a waterproof effect, ensures that the collected electrocardiogram signal has a high signal-to-noise ratio, and provides a good wearing experience for the user.

### SUMMARY

Embodiments of the present disclosure provide a device for monitoring a physiological signal. The device for monitoring a physiological signal comprises: a strap, configured to be worn on a body of a user, two electrode elements, arranged at intervals in a length direction of the strap, each of the two electrode elements including an electrode configured to contact the body of the user to collect the physiological signal, and a waterproof layer, wherein the two electrode elements are both connected to the strap through the waterproof layer. The strap includes at least an elastic yarn and an insulating yarn, the waterproof layer includes a waterproof insulating yarn, and the strap and the waterproof layer are formed by integrated weaving.

In some embodiments, the two electrode elements are located at two sides of a median sagittal plane of a human body, and the two electrode elements are configured to collect electrocardiogram signals of the human body.

In some embodiments, the electrode includes a first conductive yarn, and the electrode, the waterproof layer, and the strap are formed by integrated weaving.

In some embodiments, each of the two electrode elements includes an electrode support layer. The electrode support layer is located between the electrode and the waterproof layer. The electrode support layer at least includes a yarn different from the first conductive yarn, and the electrode support layer and the electrode are formed by integrated weaving.

In some embodiments, in a non-wearing state, an inner surface of each of the two electrode elements protrudes beyond an inner surface of the strap, and a protrusion distance is in a range of 0.1 mm to 5 mm.

In some embodiments, the waterproof layer is woven on an inner surface of the strap, and the two electrode elements are woven on a side of the waterproof layer facing away from the strap.

In some embodiments, the strap includes a first region and a second region. An elasticity of the first region is less than an elasticity of the second region. The first region is a projected region of the electrode on the strap, and the second region does not overlap with the first region.

In some embodiments, the first region does not include the elastic yarn.

In some embodiments, the strap includes a second conductive yarn, and the second conductive yarn implements electrostatic shielding for the two electrode elements.

In some embodiments, a first portion of the second conductive yarn is located on an outer surface of the strap or between the inner surface and the outer surface of the strap, such that each of the two electrode elements is at least partially located between the first portion of the second conductive yarn and the body of the user, and a second portion of the second conductive yarn extends to the inner surface of the strap and electrically connects the first portion of the second conductive yarn to the body of the user to implement the electrostatic shielding for each of the two electrode elements.

In some embodiments, the second conductive yarn is interwoven through the inner surface and the outer surface of the strap.

In some embodiments, the strap is at least formed by mixed weaving of the elastic yarn, the insulating yarn, and the second conductive yarn.

In some embodiments, each of the first portion of the second conductive yarn and the second portion of the second conductive yarn includes a plurality of conductive channels arranged in parallel in a width direction of the strap, and each conductive channel of the plurality of conductive channels is formed by weaving the second conductive yarn in a wave shape in the length direction of the strap.

In some embodiments, the two electrode elements include a first electrode element and a second electrode element that are arranged at intervals in the length direction of the strap, the first portion of the second conductive yarn forms one or more conductive regions, and the one or more conductive regions cover the first electrode element and the second electrode element.

In some embodiments, the waterproof layer and the strap are woven in a spliced manner, and the waterproof layer extends through an inner surface and an outer surface of the strap in a thickness direction of the strap.

In some embodiments, the two electrode elements are woven on an inner side of the waterproof layer.

In some embodiments, the electrode and the waterproof layer are woven in a spliced manner, and the waterproof layer is located between the strap and the two electrode elements.

In some embodiments, the strap includes a second conductive yarn, the second conductive yarn is interwoven through the inner surface and the outer surface of the strap, and the second conductive yarn implements electrostatic shielding for each of the two electrode elements.

In some embodiments, the waterproof layer divides the strap into a first sub-strap and a second sub-strap that are mutually isolated, one of the two electrode elements is located on the first sub-strap, and the other of the two electrode elements is located on the second sub-strap.

In some embodiments, a first anti-static electrode is arranged on the first sub-strap, a second anti-static electrode is arranged on the second sub-strap, and the first anti-static electrode and the second anti-static electrode cover the first conductive yarn.

In some embodiments, two connection ports are provided on the strap, the two connection ports are electrically connected to the two electrode elements, respectively, to implement data transmission between the two electrode elements and a processing device, and the processing device is detachably connected to the two connection ports by magnetic attachment.

In some embodiments, each of the two connection ports is spaced apart from yarns on the strap, and a waterproof insulating material is filled between the two connection ports and the strap.

In some embodiments, each of the two electrode elements is connected to a corresponding connection port via a conductive yarn wrapped with an insulating material.

### BRIEF DESCRIPTION OF THE DRAWINGS

The present disclosure will be further described by way of exemplary embodiments, which will be described in detail with reference to the accompanying drawings. These embodiments are not intended to be limiting. In the embodiments, the same reference numerals denote the same structures, in which:
FIG. 1 is a schematic diagram illustrating an application scenario of a device for monitoring a physiological signal according to some embodiments of the present disclosure;
FIG. 2A is a structural diagram illustrating an inner surface of a conventional device for monitoring a physiological signal according to some embodiments of the present disclosure;
FIG. 2B is a structural diagram illustrating an outer surface of a conventional device for monitoring a physiological signal according to some embodiments of the present disclosure;
FIG. 2C is a front view of a conventional device for monitoring a physiological signal according to some embodiments of the present disclosure;
FIG. 3A is a structural diagram illustrating an inner surface of a device for monitoring a physiological signal according to some embodiments of the present disclosure;
FIG. 3B is a structural diagram illustrating an outer surface of a device for monitoring a physiological signal according to some embodiments of the present disclosure;
FIG. 3C is another structural diagram illustrating an inner surface of a device for monitoring a physiological signal according to some embodiments of the present disclosure;
FIG. 3D is a front view of a device for monitoring a physiological signal according to some embodiments of the present disclosure;
FIG. 3E is a schematic diagram illustrating positions of a human body where electrode elements of a device for monitoring a physiological signal are located according to some embodiments of the present disclosure;
FIG. 3F is another front view of a device for monitoring a physiological signal according to some embodiments of the present disclosure;
FIG. 3G is yet another front view of a device for monitoring a physiological signal according to some embodiments of the present disclosure;
FIG. 3H is still another front view of a device for monitoring a physiological signal according to some embodiments of the present disclosure;
FIG. 3I is a structural diagram illustrating an inner surface of another device for monitoring a physiological signal according to some embodiments of the present disclosure;
FIG. 3J is a structural diagram illustrating an outer surface of another device for monitoring a physiological signal according to some embodiments of the present disclosure;
FIG. 3K is a front view of another device for monitoring a physiological signal according to some embodiments of the present disclosure;
FIG. 3L is another front view of another device for monitoring a physiological signal according to some embodiments of the present disclosure;
FIG. 3M is another structural diagram illustrating an inner surface of another device for monitoring a physiological signal according to some embodiments of the present disclosure;
FIG. 3N is another structural diagram illustrating an outer surface of another device for monitoring a physiological signal according to some embodiments of the present disclosure;
FIG. 3O is a schematic diagram illustrating a front projection of another device for monitoring a physiological signal according to some embodiments of the present disclosure;
FIG. 3P is a schematic diagram illustrating a side projection of another device for monitoring a physiological signal according to some embodiments of the present disclosure;
FIG. 3Q is yet another schematic diagram illustrating a front projection of another device for monitoring a physiological signal according to some embodiments of the present disclosure;
FIG. 3R is yet another schematic diagram illustrating a side projection of another device for monitoring a physiological signal according to some embodiments of the present disclosure;
FIG. 4A is a structural diagram illustrating an inner surface of yet another device for monitoring a physiological signal according to some embodiments of the present disclosure;
FIG. 4B is a structural diagram illustrating an outer surface of yet another device for monitoring a physiological signal according to some embodiments of the present disclosure;
FIG. 4C is another structural diagram illustrating an outer surface of yet another device for monitoring a physiological signal according to some embodiments of the present disclosure;
FIG. 4D is a schematic diagram illustrating a front projection of yet another device for monitoring a physiological signal according to some embodiments of the present disclosure;
FIG. 4E is a schematic diagram illustrating a side projection of yet another device for monitoring a physiological signal according to some embodiments of the present disclosure;
FIG. 4F is another schematic diagram illustrating a front projection of yet another device for monitoring a physiological signal according to some embodiments of the present disclosure;
FIG. 4G is another schematic diagram illustrating a side projection of yet another device for monitoring a physiological signal according to some embodiments of the present disclosure;
FIG. 5A is a structural diagram illustrating a surface structure of a substrate of yet another device for monitoring a physiological signal according to some embodiments of the present disclosure;
FIG. 5B is a front view of a substrate structure of yet another device for monitoring a physiological signal according to some embodiments of the present disclosure;
FIG. 5C is a structural diagram illustrating a surface structure of another substrate of yet another device for monitoring a physiological signal according to some embodiments of the present disclosure;
FIG. 5D is a front view of another substrate structure of yet another device for monitoring a physiological signal according to some embodiments of the present disclosure;
FIG. 5E is a structural diagram illustrating an inner surface of yet another device for monitoring a physiological signal according to some embodiments of the present disclosure;
FIG. 5F is a structural diagram illustrating an outer surface of yet another device for monitoring a physiological signal according to some embodiments of the present disclosure;
FIG. 5G is a front view of yet another device for monitoring a physiological signal according to some embodiments of the present disclosure;
FIG. 5H is another structural diagram illustrating an inner surface of yet another device for monitoring a physiological signal according to some embodiments of the present disclosure;
FIG. 5I is another structural diagram illustrating an outer surface of yet another device for monitoring a physiological signal according to some embodiments of the present disclosure;
FIG. 5J is another front view of yet another device for monitoring a physiological signal according to some embodiments of the present disclosure;
FIG. 5K is a structural diagram illustrating an inner surface of yet another device for monitoring a physiological signal according to some embodiments of the present disclosure;
FIG. 5L is a structural diagram illustrating an outer surface of yet another device for monitoring a physiological signal according to some embodiments of the present disclosure;
FIG. 5M is a front view of yet another device for monitoring a physiological signal according to some embodiments of the present disclosure;
FIG. 5N is another structural diagram illustrating an inner surface of yet another device for monitoring a physiological signal according to some embodiments of the present disclosure;
FIG. 5O is another structural diagram illustrating an outer surface of yet another device for monitoring a physiological signal according to some embodiments of the present disclosure;
FIG. 5P is another front view of yet another device for monitoring a physiological signal according to some embodiments of the present disclosure;
FIG. 6A is a structural diagram illustrating an inner surface of yet another device for monitoring a physiological signal according to some embodiments of the present disclosure;
FIG. 6B is a structural diagram illustrating an outer surface of yet another device for monitoring a physiological signal according to some embodiments of the present disclosure;
FIG. 6C is a front view of yet another device for monitoring a physiological signal according to some embodiments of the present disclosure;
FIG. 6D is another front view of yet another device for monitoring a physiological signal according to some embodiments of the present disclosure;
FIG. 6E is still another front view of yet another device for monitoring a physiological signal according to some embodiments of the present disclosure; and
FIG. 6F is a schematic diagram illustrating a side projection of yet another device for monitoring a physiological signal according to some embodiments of the present disclosure.

### DETAILED DESCRIPTION

In order to more clearly illustrate technical solutions of embodiments of the present disclosure, brief descriptions of the drawings to be used in the description of the embodiments are provided below. It is apparent that the drawings in the following description are merely some examples or embodiments of the present disclosure. For those skilled in the art, the present disclosure may also be applied to other similar scenarios based on these drawings without creative efforts. Unless otherwise apparent from the context or specifically stated, the same reference numerals in the drawings denote the same structures or operations.

It should be understood that the terms "system," "device," "unit," and/or "module" used herein are intended to distinguish different levels of components, elements, parts, portions, or assemblies. However, if other terms can achieve the same purpose, such expressions may be substituted for the above terms.

As used in the present disclosure and the claims, unless the context clearly indicates otherwise, the terms "a," "an," "one," and/or "the" are not intended to refer exclusively to the singular form but may also include the plural form. In general, the terms "include" and "comprise" indicate the inclusion of explicitly stated steps or elements, but such steps or elements are not an exhaustive listing, and a method or device may also include other steps or elements.

In the description of the present disclosure, it should be understood that the terms "first," "second," "third," "fourth," or the like are used merely for descriptive purposes and should not be construed as indicating or implying relative importance or implicitly specifying the number of the indicated technical features. Therefore, features designated as "first," "second," "third," or "fourth" may explicitly or implicitly include at least one of such features. In the description of the present disclosure, the term "a plurality of" means at least two, for example, two or three, unless otherwise specifically defined.

In the present disclosure, unless otherwise expressly specified or defined, the terms "connect," "fix," and the like should be understood in a broad sense. For example, the term "connect" may refer to a fixed connection, a detachable connection, or an integrated connection; may represent a mechanical connection or an electrical connection; may indicate a direct connection or an indirect connection through an intermediate medium; and may describe communication inside two elements or an interaction relationship between two elements, unless otherwise specifically limited. For those skilled in the art, the specific meanings of the above terms in the present disclosure may be understood according to the particular context.

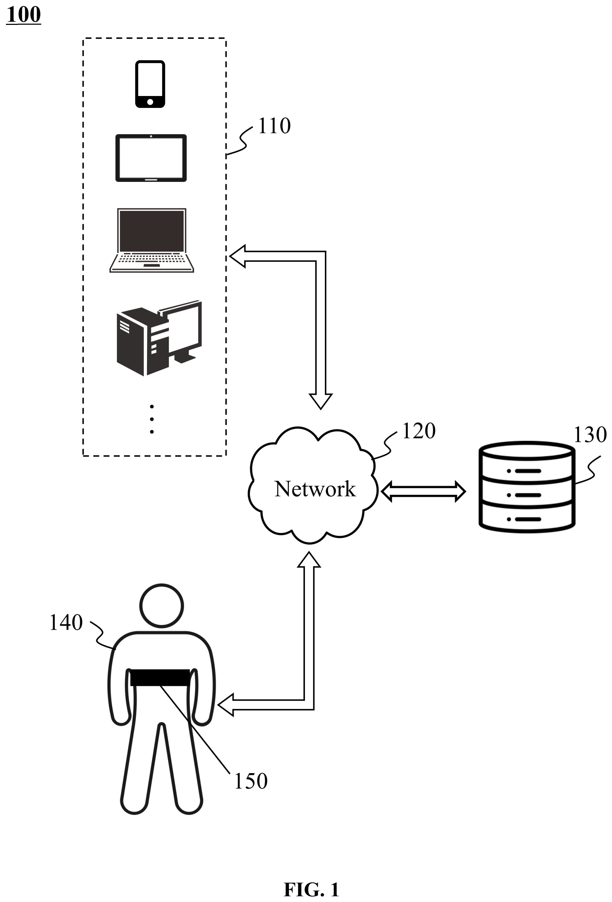

FIG. 1 is a schematic diagram illustrating an application scenario of a device for monitoring a physiological signal according to some embodiments of the present disclosure. As shown in FIG. 1, in some embodiments, an application scenario 100 of the device for monitoring a physiological signal (hereinafter referred to as the application scenario 100) may include a terminal device 110, a network 120, a storage device 130, a monitored target 140, and a device 150 for monitoring a physiological signal. In some embodiments, various components in the application scenario 100 (e.g., the terminal device 110, the storage device 130, and the device 150 for monitoring a physiological signal) may be connected and/or communicate with each other via the network 120 (e.g., a wireless connection, a wired connection, or a combination thereof).

The terminal device 110 refers to a device and/or software used by a user related to the application scenario 100. The user associated with the application scenario 100 includes, but is not limited to, the monitored target 140, physicians (e.g., clinical physicians and radiotherapy physicians), and nurses etc. For example, the terminal device 110 may be a device or software configured to control the device 150 for monitoring a physiological signal. The user may issue a control instruction to the device 150 for monitoring a physiological signal through the terminal device 110, thereby controlling the device 150 for monitoring a physiological signal to collect a physiological signal of the monitored target 140. For example, the terminal device 110 may transmit a control instruction input by the user to the device 150 for monitoring a physiological signal via the network 120 to control the device 150 for monitoring a physiological signal to perform the collection of the physiological signal of the monitored target 140. In some embodiments, the terminal device 110 may collect the physiological signal of the monitored target 140 collected by the device 150 for monitoring a physiological signal through the network 120. In some embodiments, the terminal device 110 may be one or any combination of a mobile device, a tablet computer, a laptop computer, a desktop computer, or another device having an input and/or output function.

The physiological signal refers to a bioelectrical signal (e.g., an electrocardiogram signal, an electromyogram signal, or the like) of an object (e.g., the monitored target 140) collected based on a signal collection device (e.g., the device 150 for monitoring a physiological signal). The physiological signal may be a mixed signal including both a noise signal (e.g., a motion artifact signal, an electrostatic signal, or the like) and a pure physiological signal. The pure physiological signal refers to a true physiological signal of the object (e.g., the monitored target 140) obtained after filtering out the noise signal.

The network 120 may connect various components of the application scenario 100 (e.g., the terminal device 110, the storage device 130, and the device 150 for monitoring a physiological signal) and/or connect the application scenario 100 with external resource portions. The network 120 may enable communication among the various components of the application scenario 100 and between the application scenario 100 and other portions outside the application scenario 100, thereby facilitating the exchange of data and/or information. For example, the terminal device 110 may collect the physiological signal of the monitored target 140 collected by the device 150 for monitoring a physiological signal through the network 120. As another example, the storage device 130 may collect and store physiological signal data of the monitored target 140 collected by the device 150 for monitoring a physiological signal through the network 120.

In some embodiments, the network 120 may be any form of wired network, wireless network, or any combination thereof. Merely by way of example, the network 120 may include a cable network, a wired network, a fiber-optic network, a telecommunication network, an intranet, the Internet, a local area network (LAN), a wide area network (WAN), a wireless local area network (WLAN), a metropolitan area network (MAN), a public switched telephone network (PSTN), a Bluetooth network, a ZigBee network, a near-field communication (NFC) network, or any combination thereof. In some embodiments, the network 120 may include at least one network access point, and at least one component of the application scenario 100 may be connected to the network 120 through the access point to exchange data and/or information. For example, data such as a collected physiological signal may be transmitted through the network 120.

The storage device 130 may store data, instructions, and/or any other information. In some embodiments, the storage device 130 may store data obtained from the device 150 for monitoring a physiological signal and/or the terminal device 110. For example, the storage device 130 may store the physiological signal collected by the device 150 for monitoring a physiological signal. In some embodiments, the storage device 130 may include a mass storage, a removable storage, a volatile read-write memory, a read-only memory (ROM), or any combination thereof. Exemplary mass storage may include a magnetic disk, an optical disk, a solid-state disk, etc. In some embodiments, the storage device 130 may be implemented on a cloud platform. Merely by way of example, the cloud platform may include a private cloud, a public cloud, a hybrid cloud, a community cloud, a distributed cloud, an internal cloud, a multilayer cloud, or any combination thereof.

In some embodiments, the storage device 130 may be connected to the network 120 to communicate with at least one other component in the application scenario 100. At least one component in the application scenario 100 may access data, instructions, or other information stored in the storage device 130 through the network 120. In some embodiments, the storage device 130 may be directly connected or communicate with one or more components (e.g., the device 150 for monitoring a physiological signal and the terminal device 110) in the application scenario 100. In some embodiments, the storage device 130 may be a part of the device 150 for monitoring a physiological signal and/or the terminal device 110.

The monitored target 140 refers to an object monitored by the device 150 for monitoring a physiological signal, for example, a user of the device 150 for monitoring a physiological signal or a patient or experiment participant whose physiological signal needs to be collected.

The device 150 for monitoring a physiological signal refers to a device configured to perform the collection or monitoring of the physiological signal of the monitored target 140. The device 150 for monitoring a physiological signal may be fixed to at least one body portion (e.g., a chest, a back, or a waist) of the monitored target 140 to collect the physiological signal of the monitored target 140. In some embodiments, the device 150 for monitoring a physiological signal may have a strap-shaped structure (e.g., a heart rate strap) including a strap and a plurality of electrodes (e.g., two electrodes). The strap may be configured to fix the device 150 for monitoring a physiological signal to at least one body portion of the monitored target 140. The plurality of electrodes may be arranged on an inner surface of the strap and configured to fit with at least one body portion of the monitored target 140 to collect an electrical signal (e.g., potentials at positions where the electrodes are located) of the monitored target 140.

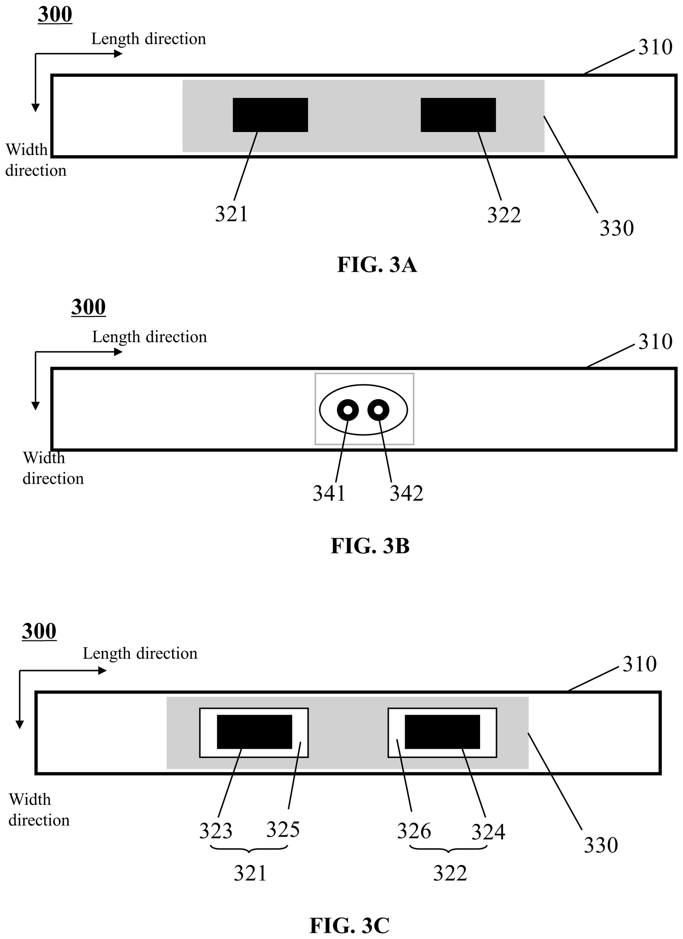

In some embodiments, the device 150 for monitoring a physiological signal may have an independent power supply. The device 150 for monitoring a physiological signal may transmit collected data (e.g., the physiological signal) to other components (e.g., the storage device 130 and the terminal device 110) in a wired or wireless manner (e.g., Bluetooth, Wi-Fi, or the like). In some embodiments, one or more components in the application scenario 100 may be a part of the device 150 for monitoring a physiological signal. For example, the device 150 for monitoring a physiological signal may include the storage device 130, etc. More descriptions regarding the device 150 for monitoring a physiological signal may be found in other contents of the present disclosure (e.g., descriptions in connection with FIGs. 3A-6F and related descriptions thereof).

In some embodiments, the application scenario 100 may further include a processing device (not shown in FIG. 1, which may be configured in the device 150 for monitoring a physiological signal or the terminal device 110). The processing device may determine and obtain physiological data based on electrical signals collected by the plurality of electrodes. The physiological data refer to data determined based on the physiological signals and reflecting bioelectrical characteristics of a monitored target (e.g., the monitored target 140). For example, when the physiological signal is the electrocardiogram signal, the physiological data may be electrocardiogram data.

It should be noted that the above descriptions regarding the application scenario 100 are merely for example and illustration, and do not limit the applicable scope of the present disclosure. For those skilled in the art, various modifications and changes to the application scenario 100 may be made under the guidance of the present disclosure. However, these modifications and changes still fall within the scope of the present disclosure.

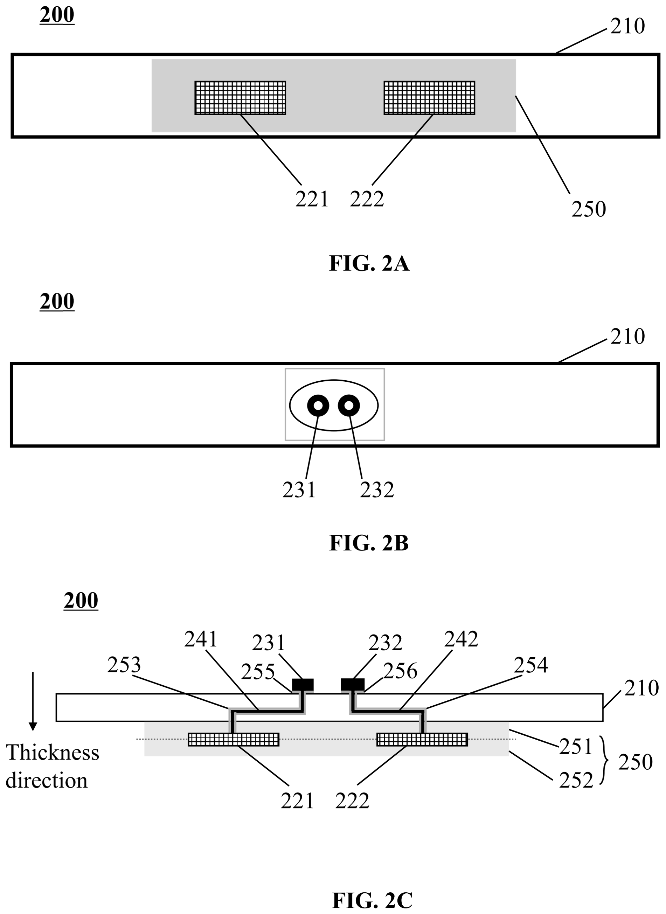

FIG. 2A is a structural diagram illustrating an inner surface of a conventional device for monitoring a physiological signal according to some embodiments of the present disclosure; FIG. 2B is a structural diagram illustrating an outer surface of the conventional device for monitoring a physiological signal according to some embodiments of the present disclosure; and FIG. 2C is a front view of the conventional device for monitoring a physiological signal according to some embodiments of the present disclosure. FIG. 2C is the front view of the conventional device for monitoring a physiological signal when the device is laid flat on a horizontal surface (e.g., when the outer surface or the inner surface of the device is placed parallel to a desktop or a floor). The same applies to other front views described below. In some embodiments, as shown in FIGs. 2A, 2B, and 2C, the conventional device 200 for monitoring a physiological signal may include a strap 210, two electrodes (an electrode 221 and an electrode 222), and two connection ports (a connection port 231 and a connection port 232).

The strap 210 serves as a substrate structure of the conventional device 200 for monitoring a physiological signal and is configured to be worn on a body of a user (e.g., the monitored target 140) such that the two electrodes fit with the body of the user. The strap 210 includes an inner surface (a contact surface with the body of the user) and an outer surface (another surface opposite to the inner surface and away from human skin). Two ends of the strap 210 may be provided with connecting members (e.g., buckles, not shown in the figure). When the connecting members at the two ends are connected (e.g., buckled), the strap 210 may surround and fit a wearing portion of the user (e.g., surround and fit a waist of the user).

The material of the strap 210 includes plant fibers (e.g., cotton fibers, linen fibers, or the like), animal fibers (e.g., wool or the like), synthetic fibers (e.g., acrylic fibers, polyester fibers, or the like), or the like. For example, the strap 210 may be woven from the plant fibers.

The electrode 221 and the electrode 222 are electrical signal collection electrodes, and the electrode 221 and the electrode 222 are not electrically connected to each other. As shown in FIG. 2A, the electrode 221 and the electrode 222 may be disposed on the inner surface of the strap 210 and fit with the body of the monitored target (e.g., the monitored target 140) to collect an electrical signal (e.g., a potential) at a position where the electrode is located.

The electrode 221 and the electrode 222 may be electrically connected to the connection port 231 and the connection port 232, respectively. For example, as shown in FIG. 2C, the electrode 221 may be electrically connected to the connection port 231 through a connecting wire 241, and the electrode 222 may be electrically connected to the connection port 232 through a connecting wire 242. The connection port 231 and the connection port 232 may be connected to a transmitter (not shown in the figure) (e.g., connected in a snap-fastening manner or connected by magnetic attraction). The transmitter may transmit electrical signals (e.g., electrocardiogram potentials) collected by the electrode 221 and the electrode 222 to a processing device, so that the processing device determines the physiological signal (e.g., the electrocardiogram signal) and/or physiological data (e.g., an electrocardiogram) of the monitored target (e.g., the monitored target 140) based on the electrical signals. In some embodiments, the connection port 231 and the connection port 232 may be directly connected to the processing device, so that the processing device may directly collect the electrical signals collected by the electrode 221 and the electrode 222 to determine the physiological signal (e.g., the electrocardiogram signal) and/or the physiological data (e.g., an electrocardiogram) of the monitored target (e.g., the monitored target 140) based on the electrical signals.

In some embodiments, as shown in FIG. 2C, the conventional device 200 for monitoring a physiological signal may be provided with a waterproof layer 250. The waterproof layer 250 includes two waterproof films (i.e., a waterproof film 251 and a waterproof film 252). The waterproof film 251 and the waterproof film 252 may be fitted together to wrap the electrode 221 and the electrode 222, thereby achieving waterproof protection for the electrode 221 and the electrode 222. When the strap 210 is wetted by sweat of the user, the strap 210 may become conductive. To prevent abnormal conduction between the electrode 221 and the electrode 222 through the strap 210 under such a condition, insulation is required between the electrode 221, the electrode 222, and the strap 210. Based on this, the material of the waterproof film 251 may be an insulating waterproof material. For example, the material of the waterproof film 251 and the waterproof film 252 may be insulating rubber, silicone, or the like.

In some embodiments, as shown in FIG. 2C, to prevent the electrodes from being electrically connected to the strap 210 through the connecting wires, the connecting wire 241 and the connecting wire 242 may be respectively wrapped with the waterproof film 253 and the waterproof film 254. The material of the waterproof film 251 may be an insulating waterproof material. For example, the material of the waterproof film 253 and the waterproof film 254 may be insulating rubber, silicone, or the like.

In some embodiments, as shown in FIG. 2C, to prevent the connection port from being electrically connected to the strap 210, an insulating layer 255 and an insulating layer 256 may be respectively provided among the connection port 231, the connection port 232, and the strap 210. The material of the insulating layer 255 and the insulating layer 256 may be an insulating waterproof material. For example, the material of the insulating layer 255 and the insulating layer 256 may be silicone or the like.

The structure of the conventional device 200 for monitoring a physiological signal described above enables the device to have a waterproof effect, thereby preventing abnormal conduction between the electrodes and ensuring that the collected physiological signal (e.g., the electrocardiogram signal) has a high signal-to-noise ratio.

However, the conventional device 200 for monitoring a physiological signal with the above structure has at least the following problems.

The conventional device 200 for monitoring a physiological signal described above is provided with a plurality of waterproof films and insulating layers, which increase the overall thickness of the device, thereby weakening overall elasticity and causing the overall adjustability and breathability of the conventional device 200 for monitoring a physiological signal to deteriorate. In addition, since the waterproof films have poor skin affinity, contact between the waterproof films and skin of a user (e.g., the monitored target 140) may cause discomfort or even skin injury to the user during wearing, thereby affecting the wearing experience of the user. The deterioration of elasticity of the conventional device 200 for monitoring a physiological signal also leads to a decrease in fitness between the conventional device 200 for monitoring a physiological signal and the human body, which adversely affects a signal-to-noise ratio of the collected physiological signal.

To achieve the waterproof effect, the conventional device 200 for monitoring a physiological signal is provided with the plurality of waterproof films (e.g., the waterproof film 251, the waterproof film 252, the waterproof film 253, and the waterproof film 254) and the insulating layers (e.g., the insulating layer 255 and the insulating layer 256), which make a manufacturing process of the device for monitoring a physiological signal complex and complicate a production procedure, thereby inevitably leading to an increase in a defect rate during production of the conventional device 200 for monitoring a physiological signal and a decrease in production capacity.

Accordingly, the embodiments of the present disclosure provide a device for monitoring the physiological signal (e.g., a device 300 for monitoring a physiological signal, a device 400 for monitoring a physiological signal, a device 500 for monitoring a physiological signal, and a device 600 for monitoring a physiological signal), which has a waterproof effect, ensures that the collected electrocardiogram signal has a high signal-to-noise ratio, and ensures a good wearing experience for the user.

FIG. 3A is a structural diagram illustrating an inner surface of a device for monitoring a physiological signal according to some embodiments of the present disclosure; and FIG. 3B is a structural diagram illustrating an outer surface of the device for monitoring a physiological signal according to some embodiments of the present disclosure.

In some embodiments, as shown in FIGs. 3A and 3B, the device 300 for monitoring a physiological signal may include a strap 310, two electrode elements (a first electrode element 321 and a second electrode element 322), and a waterproof layer 330.

The strap 310 serves as a substrate structure of the device for monitoring a physiological signal and is configured to be worn on a body of a user (e.g., the monitored target 140) (e.g., worn on the chest, back, or the waist of the user) such that the two electrode elements may fit with the body of the user. For example, two ends of the strap 310 may be provided with buckles. When the buckles at the two ends are fastened, the strap 310 may surround and fit the body of the user (e.g., surround and fit the waist of the user).

In some embodiments, a material of the strap 310 includes plant fibers (e.g., cotton fibers, linen fibers, or the like), animal fibers (e.g., wool or the like), synthetic fibers (e.g., acrylic fibers, polyester fibers, or the like), or the like. For example, the strap 310 may be woven from cotton fibers.

In some embodiments, the strap 310 may include at least an elastic yarn (e.g., a polyester yarn, a nylon yarn, or the like). That is, yarns used for weaving the strap 310 include at least the elastic yarn. For example, the strap 310 may be woven from a polyester yarn.

In some embodiments, the strap 310 may include at least the elastic yarn and an insulating yarn (e.g., a cotton yarn, a spandex yarn, or the like). That is, the yarns used for weaving the strap 310 include at least the elastic yarn and the insulating yarn. For example, the strap 310 may be woven from a mixture of the polyester yarn and the cotton yarn.

In some embodiments, the strap 310 may be formed by mixed weaving of the elastic yarn and the insulating yarn. Mixed weaving refers to a process in which a plurality of yarns are mixed according to a certain ratio (e.g., 1:5, 2:3, etc.) and then woven together into a shape.

The two electrode elements (a first electrode element 321 and a second electrode element 322) are arranged at intervals in a length direction of the strap 310. Each of the two electrode elements includes an electrode configured to contact the body of the user to collect a physiological signal (e.g., an electrocardiogram signal).

As an example, as shown in FIG. 3A, the two electrode elements include the first electrode element 321 and the second electrode element 322. The first electrode element 321 and the second electrode element 322 are arranged at intervals in the length direction of the strap 310, and both of the electrode elements are arranged on the inner surface of the strap 310. The first electrode element 321 and the second electrode element 322 each include the electrode (not shown in the figure), and the electrode is configured to receive an electrical signal from the body of the user. The inner surface of the strap 310 refers to a surface of the strap 310 that contacts the body of the user (i.e., a surface facing human skin), and the outer surface refers to another surface opposite to the inner surface of the strap 310 (i.e., a surface away from human skin).

In some embodiments, the electrode includes a first conductive yarn. The first conductive yarn refers to a yarn having conductivity. For example, the first conductive yarn may be a metal fiber yarn (e.g., a silver fiber yarn), a carbon fiber yarn, or an insulating yarn coated with a metal or another conductive material (e.g., a silver-plated yarn), or the like. Accordingly, the electrode may be woven from the first conductive yarn.

It should be noted that the electrode elements and the strap are insulated from each other, so as to ensure that the strap does not affect the collection of electrical signals from the body of the user by the electrodes, and that liquid wetting the strap does not cause the electrodes in the two electrode elements to be electrically connected. Therefore, an insulator (e.g., the waterproof layer 330) needs to be provided between the two electrode elements and the strap 310.

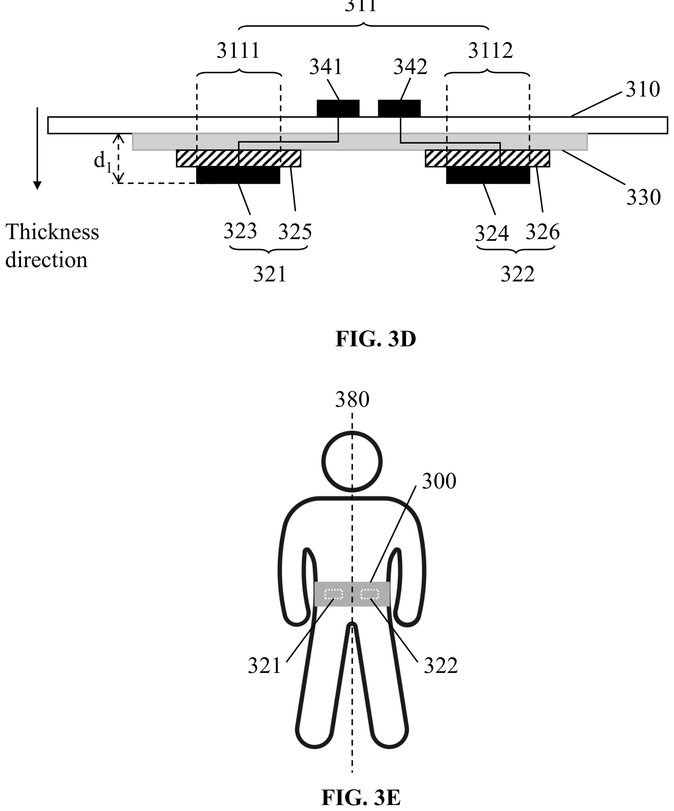

FIG. 3C is another structural diagram illustrating an inner surface of a device for monitoring a physiological signal according to some embodiments of the present disclosure; and FIG. 3D is a front view of the device for monitoring a physiological signal according to some embodiments of the present disclosure.

In some embodiments, each of the electrode elements further includes an electrode support layer, and the electrode support layer is located between the electrode and the waterproof layer. For example, as shown in FIGs. 3C and 3D, the first electrode element 321 includes a first electrode 323 and a first electrode support layer 325, and the first electrode support layer 325 is located between the first electrode 323 and the waterproof layer 330. The second electrode element 322 includes a second electrode 324 and a second electrode support layer 326, and the second electrode support layer 326 is located between the second electrode 324 and the waterproof layer 330.

The electrode support layer at least includes a yarn different from the first conductive yarn. For example, the electrode support layer may include an insulating yarn, a waterproof insulating yarn, or the like. For example, the electrode support layer may be woven from a mixture of the insulating yarn and the waterproof insulating yarn. The waterproof insulating yarn may be implemented by a variety of materials or processes. For example, a polyester yarn may be treated with a waterproofing agent to obtain the waterproof insulating yarn. No limitation is imposed herein.

FIG. 3E is a schematic diagram illustrating positions of a human body where electrode elements of a device for monitoring a physiological signal are located according to some embodiments of the present disclosure.

In some embodiments, the two electrode elements are located at two sides of a median sagittal plane of the human body, and the two electrode elements are configured to collect electrocardiogram signals of the human body. For example, taking the two electrode elements shown in FIG. 3A as an example, the first electrode element 321 and the second electrode element 322 may be respectively located at two sides of the median sagittal plane of the human body (e.g., as shown in FIG. 3E, the first electrode element 321 is located on the left side of the median sagittal plane 380 of the human body, and the second electrode element 322 is located on the right side of the median sagittal plane 380 of the human body). The median sagittal plane of the human body refers to a sagittal plane located at a middle position of the human body, and the sagittal plane passes through the umbilical midline and vertically divides the body into left and right symmetrical portions.

In some embodiments, positions on the human body where the two electrode elements fit may be symmetrical with respect to the median sagittal plane of the human body, that is, distances between the two electrode elements and the median sagittal plane of the human body are equal. Merely by way of example, taking the two electrode elements shown in FIG. 3A as an example, the first electrode element 321 is located at a position on a body surface directly opposite to a left iliac bone, and the second electrode element 322 is located at a position on the body surface directly opposite to a right iliac bone.

In some embodiments of the present disclosure, by disposing the two electrode elements on two sides of the median sagittal plane, quality of a collected electrocardiogram signal can be effectively improved, which is beneficial to improving the signal-to-noise ratio of the electrocardiogram signal. Furthermore, by further disposing the two electrode elements at symmetrical positions on the two sides of the median sagittal plane, the quality of the collected electrocardiogram signal can be further improved.

The waterproof layer 330 refers to a structure in the device 300 for monitoring a physiological signal that has waterproof and insulating functions. The two electrode elements are both connected to the strap 310 through the waterproof layer 330, that is, in a thickness direction of the strap 310, the waterproof layer 330 is located between the strap 310 and the electrode elements. When the strap 310 is wetted by a liquid (e.g., sweat, water, or the like), the waterproof layer 330 may prevent the liquid from spreading to the electrode elements, thereby achieving waterproof protection for the electrodes.

In some embodiments, the waterproof layer 330 includes a waterproof insulating yarn (e.g., an acrylic yarn, a spandex yarn, or the like), i.e., the yarns used for weaving the waterproof layer 330 include a waterproof insulating yarn. For example, the waterproof layer 330 may be woven from an acrylic yarn.

In some embodiments, two connection ports are provided on the strap, and the two connection ports are electrically connected to the two electrode elements, respectively, to implement data transmission between the electrode elements and a processing device. The processing device is detachably connected to the connection ports by magnetic attachment.

Merely by way of example, as shown in FIG. 3B, the device 300 for monitoring a physiological signal may further be provided with a connection port 341 and a connection port 342 on the outer surface of the strap 310. The first electrode element 321 and the second electrode element 322 may be electrically connected (conductively connected) to the connection port 341 and the connection port 342, respectively. The connection port 341 and the connection port 342 may be connected to a transmitter (not shown in the figure) (e.g., connected in a snap-fastening manner). The transmitter may transmit electrical signals (e.g., electrocardiogram potentials) collected by the first electrode element 321 and the second electrode element 322 to the processing device. The processing device may determine the physiological signal (e.g., the electrocardiogram signal) and/or physiological data (e.g., an electrocardiogram) of a monitored target (e.g., the monitored target 140) based on the electrical signals. The connection port 341 and the connection port 342 may also be directly connected to the processing device (not shown in the figure) (e.g., detachably connected by the magnetic attachment). The processing device may directly obtain electrical signals collected by the first electrode element 321 and the second electrode element 322 through the connection port 341 and the connection port 342 and determine the physiological signal (e.g., the electrocardiogram signal) and/or the physiological data (e.g., the electrocardiogram) of the monitored target (e.g., the monitored target 140) based on the electrical signals.

In some embodiments, each of the two connection ports is arranged at intervals from yarns on the strap, and the waterproof insulating material is filled between the two connection ports and the strap to achieve insulation between the two connection ports and the strap.

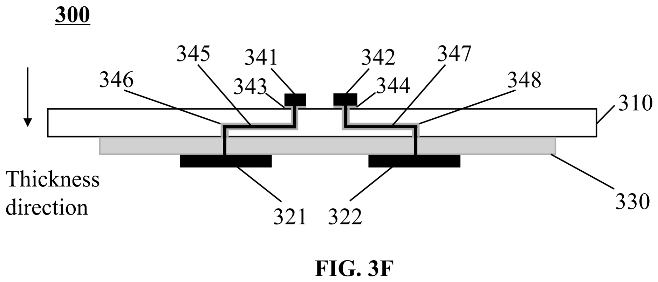

FIG. 3F is another front view of a device for monitoring a physiological signal according to some embodiments of the present disclosure.

As shown in FIG. 3F, a waterproof insulating layer 343 is filled between the connection port 341 and the strap 310, and a waterproof insulating layer 344 is filled between the connection port 342 and the strap 310, so that the connection port 341 and the connection port 342 are spaced apart and insulated from the strap 310. The waterproof insulating layer 343 and the waterproof insulating layer 344 may be made of a waterproof insulating material, for example, rubber, silicone, etc.

In some embodiments, a region of the strap that contacts the connection ports is woven using an insulating yarn to achieve insulation between the connection ports and the strap.

In some embodiments of the present disclosure, by filling the waterproof insulating material between the connection ports and the strap, liquid on the strap can be prevented from spreading to the connection ports to achieve waterproof protection of the connection ports, thereby avoiding a short circuit when the electrode elements and the processing device are connected at the connection ports.

In some embodiments, each of the two electrode elements is connected to a corresponding connection port via a conductive yarn wrapped with an insulating material.

As shown in FIG. 3F, the first electrode element 321 is connected to the connection port 341 through a conductive yarn 345, and the conductive yarn 345 may be wrapped with an insulating wrapping layer 346. The second electrode element 322 is connected to the connection port 342 through a conductive yarn 347, and the conductive yarn 347 may be wrapped with an insulating wrapping layer 348. Materials of the insulating wrapping layer 346 and the insulating wrapping layer 348 may be insulating materials, for example, rubber, plastic, etc.

In some embodiments of the present disclosure, by connecting each electrode element to the corresponding connection port through a conductive yarn wrapped with an insulating material, an electrical connection between each electrode element and the corresponding connection port can be achieved, while insulation from the strap is also maintained, thereby avoiding abnormal electrical connections between the electrodes.

In some embodiments, the strap 310 and the waterproof layer 330 may be formed by integrated weaving. The integrated weaving refers to a process of continuously weaving multiple types of yarns (e.g., the elastic yarn, the insulating yarn, and the waterproof insulating yarn) to obtain an integral fabric. Merely by way of example, the integrated weaving process of the strap 310 and the waterproof layer 330 may be as follows: based on the yarns respectively included in the strap 310 and the waterproof layer 330 (the strap 310 includes a mixed yarn of the elastic yarn and the insulating yarn, and the waterproof layer 330 includes the waterproof insulating yarn), the strap 310 and the waterproof layer 330 are continuously woven according to positional relationships of the strap 310 and the waterproof layer 330 shown in FIGs. 3A-3F to obtain a formed strap 310 and a formed waterproof layer 330. During the weaving process, when a transition between the strap 310 and the waterproof layer 330 is performed, a transition weaving technique (e.g., tuck knitting) is required. Furthermore, after the strap 310 and the waterproof layer 330 are woven, the electrodes may be fixed at preset positions on a surface of the waterproof layer 330 to complete integration of the strap, the electrodes, and the waterproof layer included in the device 300 for monitoring physiological signals. The fixing method may include: sewing the electrodes onto the surface of the waterproof layer 330 using yarns (e.g., the insulating yarn or the waterproof insulating yarn), or attaching the electrodes onto the surface of the waterproof layer 330 using an adhesive (e.g., conductive glue).

In some embodiments, the electrodes, the waterproof layer 330, and the strap 310 may be formed by the integrated weaving. During the process of the integrated weaving, a weaving machine replaces yarns used for weaving other surrounding regions (e.g., the waterproof layer 330) with conductive yarns when weaving the electrodes.

In some embodiments, the waterproof layer 330 may be woven on the inner surface of the strap 310, and the electrode elements (including the first electrode element 321 and the second electrode element 322) may be woven on a side of the waterproof layer 330 facing away from the strap 310.

Merely by way of example, taking the structure of the device 300 for monitoring a physiological signal shown in FIG. 3A as an example, when each electrode element does not include an electrode support layer, an integrated weaving process of the electrodes (in this case, each electrode element only includes an electrode), the strap 310, and the waterproof layer 330 may be as follows: a strap 310 with a first preset thickness is formed by mixed weaving using the elastic yarn and the insulating yarn; a waterproof layer 330 with a second preset thickness is formed by weaving the waterproof insulating yarn in a preset waterproof region on the inner surface of the strap 310; and within a preset electrode region, electrodes with a third preset thickness is formed by weaving the electrodes on the inner surface of the waterproof layer 330 using the first conductive yarn. The first preset thickness refers to a preset thickness of the strap 310, and the value of the first preset thickness may be preset (e.g., 1.5 mm, 1.8 mm, or 2 mm). The second preset thickness refers to a preset thickness of the waterproof layer 330, and the value of the second preset thickness may be preset (e.g., 1 mm, 1.2 mm, or 1.5 mm). The third preset thickness refers to a preset thickness of the electrodes, and the value of the third preset thickness may be preset (e.g., 0.4 mm, 0.5 mm, or 0.6 mm). The preset waterproof region refers to a region on the surface of the strap that carries the waterproof layer (e.g., a region where the waterproof layer 330 is located, as shown in FIG. 3A). The preset electrode region refers to a region on the surface of the waterproof layer that carries the electrode elements, for example, regions where the first electrode element 321 and the second electrode element 322 are located as shown in FIG. 3A. When each electrode element includes an electrode support layer, an integrated weaving process of the electrode elements, the strap 310, and the waterproof layer 330 may be found in the following descriptions.

It can be understood that since diameters of yarns are generally at a micron level, the strap 310 obtained through the above weaving process may include a plurality of layers of the elastic yarns and the insulating yarns, the waterproof layer 330 may include a plurality of layers of the waterproof insulating yarns, and the electrode may include a plurality of layers of the first conductive yarns to form corresponding thicknesses.

In some embodiments of the present disclosure, by weaving the electrode elements on the side of the waterproof layer facing away from the strap, the waterproof layer can prevent liquid in the strap from spreading to the electrodes, thereby achieving waterproof protection of the electrodes.

In some embodiments of the present disclosure, by integrally weaving the electrodes, the waterproof layer, and the strap, a process flow of the device for monitoring a physiological signal can be simplified, and a process duration can be shortened, which facilitates mass production of the device for monitoring a physiological signal. In addition, the electrodes and the waterproof layer formed by weaving different yarns have better skin affinity and comfort compared with those formed by traditional methods.

In some embodiments, when the electrode element includes an electrode support layer, the electrode support layer and the electrode may be formed by integrated weaving. Merely by way of example, the integrated weaving process of the electrode support layer and the electrode may be as follows: the electrode support layer with a fourth preset thickness is formed by mixed weaving using the insulating yarn and the waterproof insulating yarn; the electrode with the third preset thickness is formed by weaving the first conductive yarn in a preset electrode region on one side surface of the electrode support layer to obtain the electrode element. On this basis, the electrode element may be fixed at a preset electrode position on the inner surface of the waterproof layer 330 to complete integration of the strap 310, the electrode element, and the waterproof layer 330 included in the device 300 for monitoring a physiological signal. The fixing manner may include: sewing the electrode element onto the inner surface of the waterproof layer 330 using a yarn (e.g., the insulating yarn or the waterproof insulating yarn), or attaching the electrode element onto the inner surface of the waterproof layer 330 using an adhesive (e.g., conductive glue). The fourth preset thickness refers to a preset thickness of the electrode support layer, and the value of the fourth preset thickness may be preset, for example, 0.8 mm, 1 mm, or 1.5 mm.

In some embodiments, when the electrode element includes the electrode support layer, the electrode element (including the electrode support layer and the electrode), the waterproof layer 330, and the strap 310 may be formed by the integrated weaving. Merely by way of example, the integrated weaving process of the strap 310 and the waterproof layer 330 may be as follows: the strap 310 with the first preset thickness is formed by mixed weaving using the elastic yarn and the insulating yarn; the waterproof layer 330 with the second preset thickness is formed by weaving the waterproof insulating yarn in the preset waterproof region on the inner surface of the strap 310; within a preset electrode region, the electrode support layer with the fourth preset thickness is formed by mixed weaving using the insulating yarn and the waterproof insulating yarn; the electrode is formed by weaving the first conductive yarn on a surface of the electrode support layer, where an area of the electrode is not greater than an area of the electrode support layer, to complete integration of the strap, the electrode element (including the electrode support layer and the electrode), and the waterproof layer included in the device 300 for monitoring a physiological signal.

It can be understood that when the electrode element includes the electrode support layer, the electrode can protrude significantly beyond the inner surfaces of the strap and the waterproof layer, allowing the electrode to better fit the human body when the user wears the device for monitoring a physiological signal. The inner surface of the waterproof layer refers to a surface of the waterproof layer facing away from the inner surface of the strap (correspondingly, the outer surface of the waterproof layer refers to a surface of the waterproof layer that fits or is embedded into the inner surface of the strap).

In some embodiments, when the electrode element does not include an electrode support layer, the thickness of the electrode may be a sum of the third preset thickness and the fourth preset thickness, so that the electrode can protrude significantly beyond the inner surfaces of the strap and the waterproof layer.

In some embodiments, in a non-wearing state, the inner surface of each of the two electrode elements protrudes beyond the inner surface of the strap, and a protrusion distance is in a range of 0.1 mm to 5 mm. The non-wearing state refers to a natural state of the device for monitoring a physiological signal when the device is not worn by the user. The inner surface of the electrode element refers to a surface of the electrode element that contacts the body of the user (i.e., a surface of the electrode that contacts the body of the user).

The protrusion distance refers to a distance between the inner surface of the electrode element and the inner surface of the strap in the thickness direction of the strap. As shown in FIG. 3D, d₁ represents the protrusion distance.

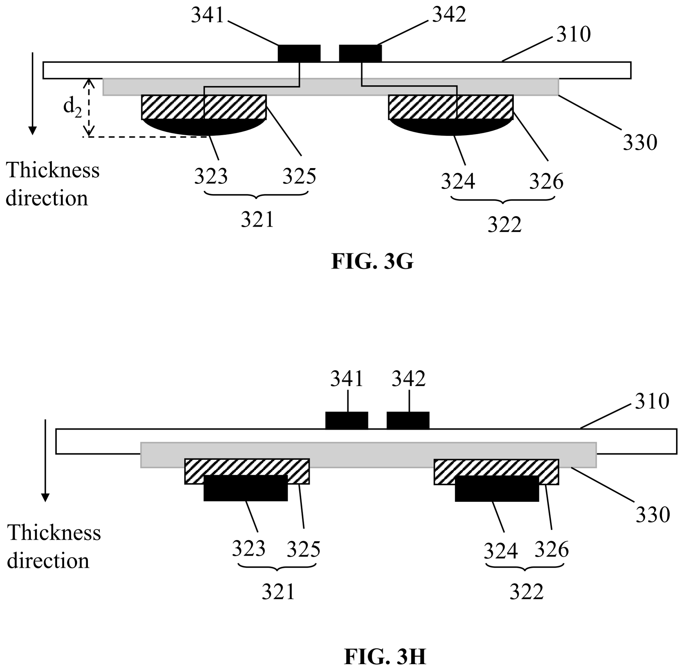

FIG. 3G is yet another front view of a device for monitoring a physiological signal according to some embodiments of the present disclosure.

In some embodiments, when the inner surface of the electrode element is non-planar, the protrusion distance refers to a maximum distance between the inner surface of the electrode element and the inner surface of the strap in the thickness direction of the strap. As shown in FIG. 3G, the inner surface of the electrode element is non-planar, and d₂ represents the protrusion distance.

In some embodiments of the present disclosure, the electrode element including the electrode support layer can ensure that the electrode significantly protrudes beyond the strap and the waterproof layer, so that when a user wears the device for monitoring a physiological signal, the electrode can fit the human body, enhancing stability of physiological signal collection and thereby improving a signal-to-noise ratio of the collected physiological signal. By providing the protrusion distance, close contact between the electrode and the human body can be ensured.

In some embodiments, during the integrated weaving of the strap 310, the electrode element, and the waterproof layer 330, the waterproof layer 330 may be embedded in the strap 310 and/or the electrode element may be embedded in the waterproof layer 330, indicating that projections of the waterproof layer 330 and the strap 310, or projections of the electrode element and the waterproof layer 330, at least partial overlap in the thickness direction of the strap 310.

FIG. 3H is still another front view of a device for monitoring a physiological signal according to some embodiments of the present disclosure.

As shown in FIG. 3H, during the integrated weaving, in the thickness direction of the strap 310, a portion of the waterproof layer 330 may be embedded in the strap 310, and a portion of the electrode elements (including the first electrode element 321 and the second electrode element 322) may be embedded in the waterproof layer 330. Furthermore, as shown in FIG. 3H, when each electrode element includes an electrode support layer (the first electrode element 321 includes the first electrode 323 and the first electrode support layer 325, and the second electrode element 322 includes the second electrode 324 and the second electrode support layer 326), a portion of an electrode may also be embedded in a corresponding electrode support layer (a portion of the first electrode 323 is embedded in the first electrode support layer 325, and a portion of the second electrode 324 is embedded in the second electrode support layer 326), thereby forming the structure of the device 300 for monitoring a physiological signal as shown in FIG. 3H.

In some embodiments, by embedding the waterproof layer in the strap, embedding the electrode elements in the waterproof layer, and even embedding the electrode in the electrode support layer, an overall thickness of the device for monitoring a physiological signal can be reduced, which is beneficial for improving wearing comfort of a user.

In some embodiments, the strap 310 includes a first region and a second region, an elasticity of the first region is less than an elasticity of the second region. The first region is a projected region of the electrodes on the strap 310, and the second region does not overlap with the first region. Merely by way of example, as shown in FIG. 3D, the projected region of the first electrode 323 on the strap 310 is a first sub-region 3111, and the projected region of the second electrode 324 on the strap 310 is a second sub-region 3112. The first sub-region 3111 and the second sub-region 3112 together form a first region 311. Based on this, the second region is located in other portions of the strap 310, except the first region 311, for example, a portion on the strap 310 that are not covered by the waterproof layer 330.

In some embodiments, to make the elasticity of the first region smaller than the elasticity of the second region, the density of the elastic yarn in the first region is smaller than the density of the elastic yarn in the second region. The density of the elastic yarn refers to a proportion of the elastic yarn used for weaving the strap 310. For example, when a ratio of the elastic yarn to other yarns (e.g., the insulating yarn) used for weaving the strap 310 is 1:4, the density of the elastic yarn is 20%. In some embodiments, the density of the elastic yarn in the first region is less than 70%. In some embodiments, the density of the elastic yarn in the second region is greater than 30%.

In some embodiments, to make the elasticity of the first region smaller than the elasticity of the second region, the elasticity of the elastic yarn in the first region is greater than the elasticity of the elastic yarn in the second region. For example, the elastic yarn in the first region may be a nylon yarn, and the elastic yarn in the second region may be a polyester yarn (the elasticity of nylon is greater than that of polyester).

In some embodiments, to make the elasticity of the first region smaller than the elasticity of the second region, the elastic yarn in the first region is tightened during weaving of the first region, while the elastic yarn in the second region is not tightened during weaving of the second region (i.e., the elastic yarn in the second region are kept in a relatively loose state).

In some embodiments, the first region does not include the elastic yarn, i.e., the density of the elastic yarn in the first region is 0.

It should be noted that since the positional relationship between the electrodes and the strap is relatively fixed, when the user wears the device for monitoring a physiological signal and performs movement, the elasticity of the strap affects a degree of deformation of the electrodes, thereby influencing the signal-to-noise ratio of the physiological signals collected by the electrodes. For example, when the elasticity of an electrode region (that is, the first region) on the strap is relatively large, the electrode region is prone to deformation, which causes deformation of the electrodes located in the electrode region, thereby affecting a quality of the physiological signals collected by the electrodes and resulting in a reduction in the signal-to-noise ratio. Moreover, the elasticity of the strap is mainly affected by the density of the elastic yarn in the strap. The greater the density of the elastic yarn is, the stronger the elasticity of the strap becomes, and the strap is more likely to deform. Accordingly, in some embodiments of the present disclosure, the elasticity of the electrode region is reduced by setting a density of the elastic yarn in the electrode region smaller than a density of the elastic yarn in a non-electrode region (that is, the second region), thereby reducing the decrease in the signal-to-noise ratio caused by deformation of the electrodes. Furthermore, by setting the density of the elastic yarn in the electrode region to 0, the elasticity of the electrode region can be further reduced.

In some embodiments, the strap includes a second conductive yarn, and the second conductive yarn implements electrostatic shielding for the two electrode elements.

The second conductive yarn refers to a yarn having electrical conductivity. For example, the second conductive yarn may be a metal fiber yarn (e.g., a silver fiber yarn), a carbon fiber yarn, or an insulating yarn coated with metal or another conductive material (e.g., a silver-plated yarn), etc.

The electrostatic shielding refers to a state in which the electrode elements of the device for monitoring a physiological signal are not affected by external charges or electric fields (e.g., electrostatic fields). For example, based on the structure of the device 300 for monitoring a physiological signal as shown in FIG. 3A, when static electricity exists on the outer surface of the strap 310, static charges may sequentially pass through a portion of the second conductive yarn located on the outer surface of the strap 310, a portion of the second conductive yarn located inside the strap 310, and a portion of the second conductive yarn located on the inner surface of the strap 310 and then enter the human body, such that the static charges cannot affect the first electrode element 321 and the second electrode element 322.

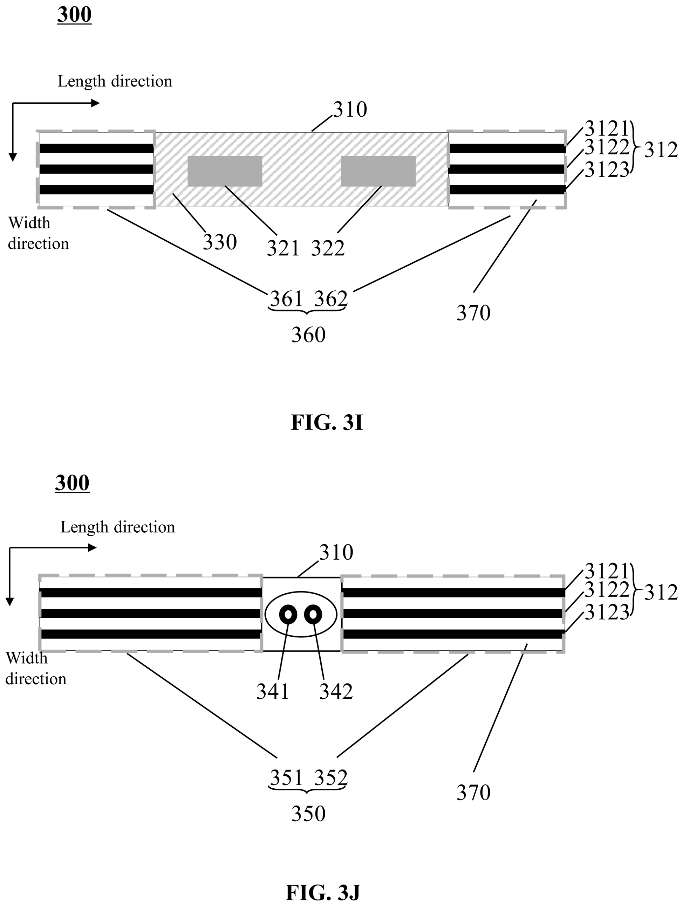

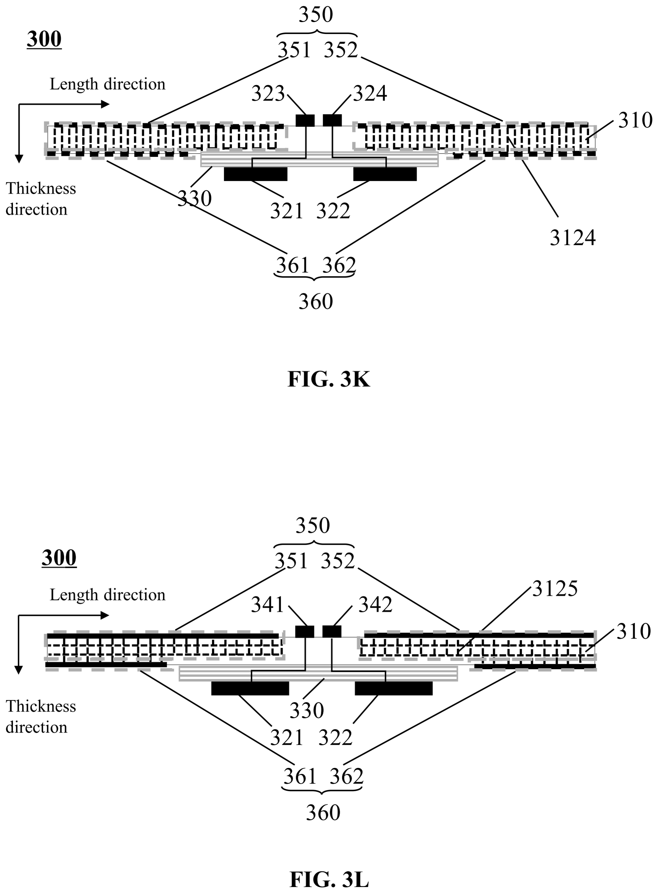

FIG. 3I is a structural diagram illustrating an inner surface of another device for monitoring a physiological signal according to some embodiments of the present disclosure; FIG. 3J is a structural diagram illustrating an outer surface of another device for monitoring a physiological signal according to some embodiments of the present disclosure; FIG. 3K is a front view of another device for monitoring a physiological signal according to some embodiments of the present disclosure; and FIG. 3L is another front view of another device for monitoring a physiological signal according to some embodiments of the present disclosure;

In some embodiments, as shown in FIGs. 3I-3L, the device 300 for monitoring a physiological signal may include the strap 310, two electrode elements (the first electrode element 321 and the second electrode element 322), and the waterproof layer 330. The strap 310 is provided with one or more strands of second conductive yarns 312 (e.g., a first strand of second conductive yarn 3121, a second strand of second conductive yarn 3122, and a third strand of second conductive yarn 3123). A first portion 350 of the second conductive yarns 312 is located on the outer surface of the strap 310 or between the inner surface and the outer surface of the strap 310, such that each electrode element is at least partially located between the first portion 350 of the second conductive yarns 312 and the body of the user. A second portion 360 of the second conductive yarns 312 (including a left second portion 361 and a right second portion 362) extends to the inner surface of the strap 310 and electrically connects the first portion 350 of the second conductive yarns 312 to the body of the user to implement electrostatic shielding for each electrode element.

In some embodiments, the strap 310 is at least formed by mixed weaving of the elastic yarn (e.g., a polyester yarn) and the insulating yarn (e.g., a spandex yarn), and the second conductive yarns 312 are interwoven through the inner surface and the outer surface of the strap 310. As shown in FIGs. 3I and 3J, the strap 310 may include an elastic portion 370 (a white portion in the figure) composed of the elastic yarn and the insulating yarn, and a plurality of strands of the second conductive yarns 312 (black portions in the figure, including the first strand of second conductive yarn 3121, the second strand of second conductive yarn 3122, and the third strand of second conductive yarn 3123).

In some embodiments, as shown in FIG. 3K, the second conductive yarns 312 include a plurality of strands (e.g., the first strand of second conductive yarn 3121, the second strand of second conductive yarn 3122, and the third strand of second conductive yarn 3123), which may be interwoven through the inner surface and the outer surface of the strap 310 to form the first portion 350 and the second portion 360 of the second conductive yarns 312. In some embodiments, the first portion 350 includes portions of the second conductive yarns 312 located on the outer surface of the strap 310 and portions located between the outer surface and the inner surface of the strap 310 (e.g., including portions of the first strand of second conductive yarn 3121, the second strand of second conductive yarn 3122, and the third strand of second conductive yarn 3123 that are located on the outer surface of the strap 310 and between the outer surface and the inner surface of the strap 310). The second portion 360 includes portions located on the inner surface of the strap 310 (e.g., including portions of the first strand of second conductive yarn 3121, the second strand of second conductive yarn 3122, and the third strand of second conductive yarn 3123 that are located on the inner surface of the strap 310). The portions of the second conductive yarns 312 located between the inner surface and the outer surface of the strap 310 are referred to as a first internal conductive yarn, for example, the first internal conductive yarn 3124 in FIG. 3K. An internal conductive yarn portion of the second conductive yarns 312 (e.g., the first internal conductive yarn 3124) may electrically connect the portions of the second conductive yarns 312 located on the inner surface of the strap 310 and those located on the outer surface of the strap 310 to implement the electrostatic shielding.

To implement the structure of the device 300 for monitoring a physiological signal described in the above embodiments, the weaving process of the device 300 for monitoring a physiological signal may include: first, weaving the strap 310 based on the elastic yarn and the insulating yarn, and then interweaving a plurality of strands of the second conductive yarns 312 (e.g., the first strand of second conductive yarn 3121, the second strand of second conductive yarn 3122, and the third strand of second conductive yarn 3123) through the inner surface and the outer surface of the strap 310, respectively.

The specific process of the interweaving described above may include: weaving in parallel the plurality of strands of the second conductive yarns 312. For example, the first strand of second conductive yarn 3121, the second strand of second conductive yarn 3122, and the third strand of second conductive yarn 3123 are interwoven in the length direction of the strap 310 with a certain spacing (either equal or unequal spacing) in the width direction of the strap 310. Each strand of the conductive yarn has a sufficient length to extend from one end to the other end of the strap 310 in the length direction. As a result, the second conductive yarns 312, including three strands arranged in parallel at intervals in the width direction of the strap 310, i.e., the first strand of second conductive yarn 3121, the second strand of second conductive yarn 3122, and the third strand of second conductive yarn 3123 that are arranged in parallel at intervals in the width direction of the strap 310, are obtained.

According to the structure of the device 300 for monitoring a physiological signal as shown in FIG. 3K, when static electricity is present on the outer surface of the strap 310, static charges may sequentially pass through the first portion 350 of the second conductive yarns 312 (a portion located on the outer surface of the strap 310, e.g., a portion of the first strand of second conductive yarn 3121 located on the outer surface of the strap 310, the first internal conductive yarn 3124), and the second portion 360 (a portion of the second conductive yarn 312 located on the inner surface of the strap 310, e.g., a portion of the first strand of second conductive yarn 3121 located on the inner surface of the strap 310) into the human body, thereby preventing the static charges from affecting the first electrode element 321 and the second electrode element 322.

In some embodiments of the present disclosure, using an elastic yarn to weave the strap can ensure that the strap has good elasticity and softness, thereby improving the wearing comfort for the user. By interweaving the conductive yarns (the second conductive yarns) through the inner and the outer surfaces of the strap, the conductive yarns can form a conductive region on the outer surface to dissipate static electricity and guide the static electricity into the human body, thereby achieving the electrostatic shielding for the electrodes.

In some embodiments, the strap 310 may be at least formed by mixed weaving of the elastic yarn, the insulating yarn, and the second conductive yarn. Mixed weaving refers to a process in which the elastic yarn, the insulating yarn, and the second conductive yarn are combined into an integral structure in a certain proportion (e.g., 1:3:5, 1:2:3, etc.) and integrally woven to form the strap 310.

As shown in FIG. 3L, based on the mixed weaving manner of the strap 310 described above, the second conductive yarns 312 (e.g., the first strand of second conductive yarn 3121, the second strand of second conductive yarn 3122, and the third strand of second conductive yarn 3123) may include the first portion 350 and the second portion 360. The first portion 350 includes the portions of the second conductive yarns 312 located on the outer surface of the strap 310 and portions located between the outer surface and the inner surface of the strap 310 (e.g., the portions of the first strand of second conductive yarn 3121, the second strand of second conductive yarn 3122, and the third strand of second conductive yarn 3123 located on the outer surface of the strap 310 and portions located between the outer surface and the inner surface of the strap 310). The second portion 360 includes the portions located on the inner surface of the strap 310 (e.g., the portions of the first strand of second conductive yarn 3121, the second strand of second conductive yarn 3122, and the third strand of second conductive yarn 3123 located on the inner surface of the strap 310). The portions of the second conductive yarns 312 located between the inner surface and the outer surface of the strap 310 are referred to as a second internal conductive yarn, for example, a second internal conductive yarn 3125 as shown in FIG. 3L. An internal conductive yarn portion of the second conductive yarns 312 (e.g., the second internal conductive yarn 3125) may electrically connect the portions of the second conductive yarns 312 located on the inner surface of the strap 310 with the portions located on the outer surface of the strap 310. Each internal conductive yarn corresponds to one conductive channel, and each conductive channel can independently electrically connect the portions of the second conductive yarns 312 located on the inner surface of the strap 310 with the portions located on the outer surface of the strap 310, thereby implementing the electrostatic shielding.

In some embodiments of the present disclosure, when a count of conductive channels is small, a break in the conductive yarn at the conductive channel may affect the electrostatic shielding performance. In the mixed weaving manner of the elastic yarn, the insulating yarn, and the conductive yarns (i.e., the second conductive yarns), since the conductive yarns are distributed among multiple layers of the strap (the weaving process forms a multilayer laminar structure during the weaving of the strap), the count of conductive channels between the inner surface and the outer surface is effectively increased. As a result, static electricity on the outer surface can be better guided into the human body, thereby improving the stability of the electrostatic shielding.

In some embodiments, each of the first portion and the second portion of the second conductive yarns includes a plurality of conductive channels arranged in parallel in the width direction of the strap.

The conductive channel refers to a channel through which charges (e.g., static charges) may flow. For example, each strand of second conductive yarn in the strap may correspond to one conductive channel. The count of conductive channels may be preset by a designer or manufacturer of the device for monitoring a physiological signal, and during manufacturing of the device for monitoring a physiological signal, a preset count of conductive yarns may be woven into the strap to obtain the corresponding count of conductive channels. Merely by way of example, the count of conductive channels may be three, four, five, etc.

Merely by way of example, as shown in FIGs. 3I and 3J, both the first portion 350 and the second portion 360 of the second conductive yarns 312 include three strands of second conductive yarns (the first strand of second conductive yarn 3121, the second strand of second conductive yarn 3122, and the third strand of second conductive yarn 3123) arranged in parallel in the width direction of the strap 310. The first strand of second conductive yarn 3121, the second strand of second conductive yarn 3122, and the third strand of second conductive yarn 3123, respectively, correspond to one conductive channel. Based on this, both the first portion 350 and the second portion 360 of the second conductive yarns 312 include three conductive channels arranged in parallel in the width direction of the strap 310.

In some embodiments of the present disclosure, the conductive yarns (i.e., the second conductive yarns) form a plurality of conductive channels arranged in parallel in the width direction of the strap, and each conductive channel of the plurality of conductive channels may guide static electricity on the outer surface into the human body, thereby implementing the electrostatic shielding for the electrodes.

In some embodiments, each conductive channel of the plurality of conductive channels is formed by weaving a conductive yarn in a wave shape in the length direction of the strap.

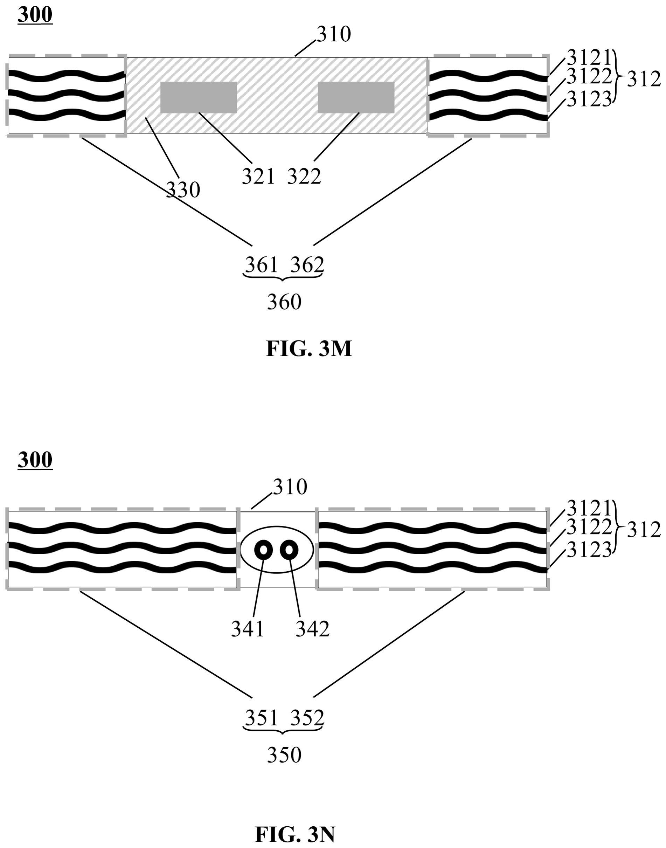

FIG. 3M is another structural diagram illustrating an inner surface of another device for monitoring a physiological signal according to some embodiments of the present disclosure; and FIG. 3N is another structural diagram illustrating an outer surface of another device for monitoring a physiological signal according to some embodiments of the present disclosure.

As shown in FIGs. 3M and 3N, each strand of second conductive yarn 312 in the strap 310 (e.g., the first strand of second conductive yarn 3121, the second strand of second conductive yarn 3122, and the third strand of second conductive yarn 3123) is woven in a wave shape in the length direction of the strap 310. Based on this, the conductive channels corresponding to the second conductive yarns 312 are curved in a wave shape in the length direction of the strap 310.

In some embodiments, each conductive channel of the plurality of conductive channels may further have a shape of a Z-shaped line, a right-angle wave line, or the like in the length direction of the strap, without limitation thereto.

In some embodiments of the present disclosure, weaving the conductive yarn (or the conductive channel) in a wave shape may reserve a stretching displacement for the conductive yarn, ensuring that the wave-shaped conductive channel formed by the conductive yarn is stretchable in the length direction, making the conductive yarn less likely to break when the strap is stretched, thereby ensuring that the strap as a whole has good elasticity and extensibility.

In some embodiments, the two electrode elements include a first electrode element and a second electrode element arranged at intervals in the length direction of the strap, the first portion of the second conductive yarns forms one or more conductive regions, and the one or more conductive regions cover the first electrode element and the second electrode element.

In some embodiments, as shown in FIG. 3I, the two electrode elements include the first electrode element 321 and the second electrode element 322 that are arranged at intervals in the length direction of the strap 310. In some embodiments, as shown in FIG. 3J, the first portion 350 of the second conductive yarns 312 forms a first conductive region 351 and a second conductive region 352 that are insulated from each other, the first conductive region 351 covering an outer side of the first electrode element 321 and being insulated from the first electrode element 321, and the second conductive region 352 covering an outer side of the second electrode element 322 and being insulated from the second electrode element 322.

The covering refers to a projected region of the electrode element being contained within a projected region of the corresponding conductive region. The projected region refers to a region corresponding to a projection of the conductive region or the electrode element in the thickness direction of the strap. In some embodiments, considering that conductive yarns in the conductive region form a plurality of conductive channels arranged in parallel and spaced apart from each other, rather than being continuously distributed throughout the entire conductive region, the conductive region "covering" the outer side of the electrode element refers to that: the projection of the conductive region has two boundaries farthest apart from each other in the length direction (or the width direction) of the strap (e.g., two points farthest apart in the length direction or the width direction), and the projected region of the electrode element in the length direction (or the width direction) is located between the two boundaries.

In some embodiments, the first conductive region covers the first electrode element in the length direction of the strap, and the second conductive region covers the second electrode element in the length direction of the strap.

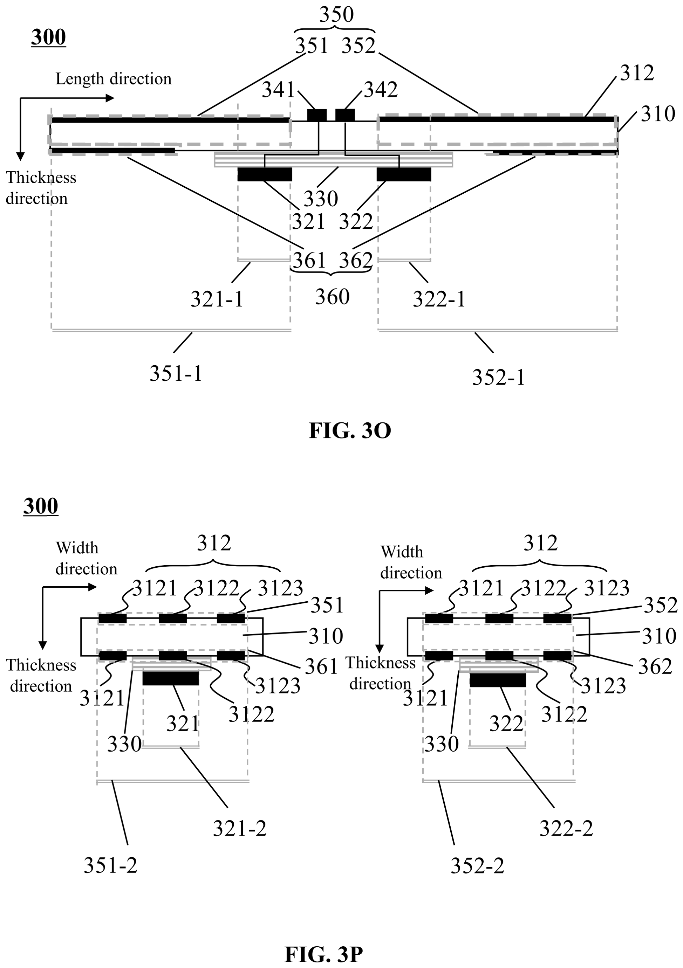

FIG. 3O is a schematic diagram illustrating a front projection of another device for monitoring a physiological signal according to some embodiments of the present disclosure.

Merely by way of example, as shown in FIG. 3O, a projected long side 321-1 of the first electrode element 321 (a side of the projected region of the first electrode element 321 that is parallel to the length direction of the strap 310) is contained within a projected long side 351-1 of the first conductive region 351 (a side of the projected region of the first conductive region 351 that is parallel to the length direction of the strap 310), and a projected long side 322-1 of the second electrode element 322 (a side of the projected region of the second electrode element 322 that is parallel to the length direction of the strap 310) is contained within a projected long side 352-1 of the second conductive region 352 (a side of the projected region of the second conductive region 352 that is parallel to the length direction of the strap 310), so that the first conductive region 351 covers the first electrode element 321 in the length direction of the strap 310, and the second conductive region 352 covers the second electrode element 322 in the length direction of the strap 310. Taking the projected long side 321-1 of the first electrode element 321 being contained within the projected long side 351-1 of the first conductive region 351 as an example, the term "contained within" refers to that the length of the projected long side 321-1 of the first electrode element 321 is not greater than the length of the projected long side 351-1 of the first conductive region 351, and the projected long side 321-1 of the first electrode element 321 is located within the projected long side 351-1 of the first conductive region 351 in spatial position. The containment relationship described later follows the same principle.

In some embodiments, by arranging the conductive region to cover the corresponding electrode element in the length direction, the conductive region may disperse static electricity near the outer surface directly facing the electrode element, thereby minimizing or eliminating the influence of the static electricity on the electrode.

In some embodiments, the first conductive region covers the first electrode element in the width direction of the strap, and the second conductive region covers the second electrode element in the width direction of the strap 310.

FIG. 3P is a schematic diagram illustrating a side projection of another device for monitoring a physiological signal according to some embodiments of the present disclosure. The left and right diagrams in FIG. 3P are schematic side projection views observed from two opposite sides of the device 300 for monitoring a physiological signal.

Merely by way of example, as shown in FIG. 3P, a projected width edge 321-2 of the first electrode element 321 (a side of the projected region of the first electrode element 321 that is parallel to the width direction of the strap 310) is contained within a projected width edge 351-2 of the first conductive region 351 (a side of the projected region of the first conductive region 351 that is parallel to the width direction of the strap 310). Similarly, a projected width edge 322-2 of the second electrode element 322 (a side of the projected region of the second electrode element 322 that is parallel to the width direction of the strap 310) is contained within a projected width edge 352-2 of the second conductive region 352 (a side of the projected region of the second conductive region 352 that is parallel to the width direction of the strap 310), such that the first conductive region 351 covers the first electrode element 321 in the width direction of the strap 310, and the second conductive region 352 covers the second electrode element 322 in the width direction of the strap 310.

In some embodiments of the present disclosure, by arranging the conductive region to cover the corresponding electrode element in the width direction, the conductive region may disperse static electricity near the outer surface directly facing the electrode element, thereby minimizing or eliminating the influence of the static electricity on the electrode.

In some embodiments of the present disclosure, the static electricity that has the greatest impact on the electrode is the static electricity located on and near the outer surface directly facing the electrode element. By arranging the conductive region to cover the corresponding electrode element, the conductive region may disperse the static electricity near the outer surface directly facing the electrode element, thereby minimizing or eliminating the influence of the static electricity on the electrode.

In some embodiments, the first portion 350 of the second conductive yarns 312 may form one conductive region, and the conductive region covers the first electrode element 321 and the second electrode element 322 in the length direction of the strap. In conjunction with FIG. 3J, the first conductive region 351 and the second conductive region 352 corresponding to the first portion 350 of the second conductive yarns 312 may be electrically connected to form one conductive region corresponding to the first portion 350. The conductive region covers the first electrode element 321 and the second electrode element 322 in the length direction of the strap 310.

In some embodiments, the conductive region corresponding to the first portion 350 of the second conductive yarns 312 covers the first electrode element 321 and the second electrode element 322 in the length direction.

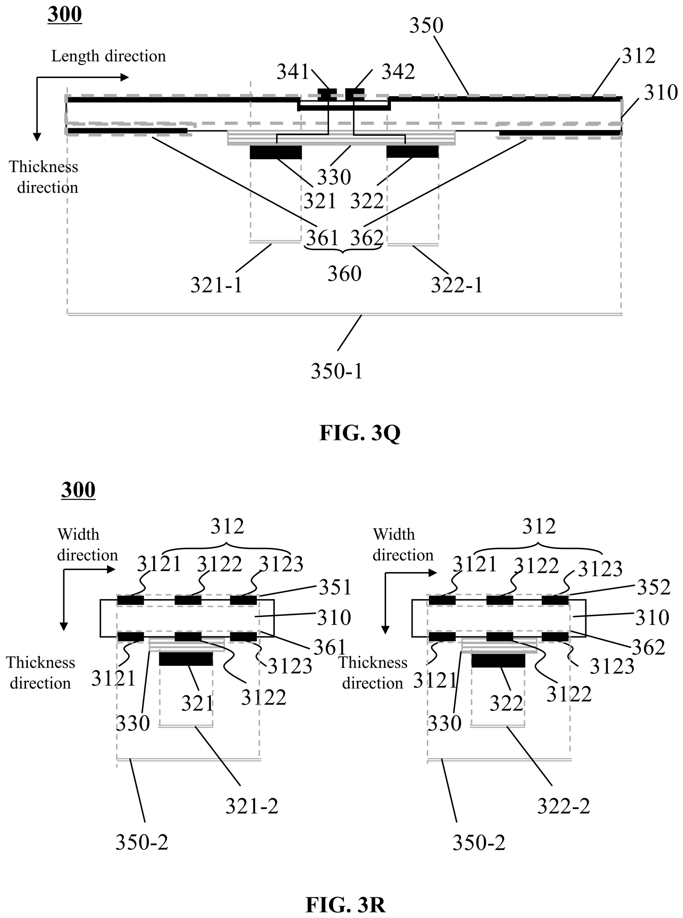

FIG. 3Q is yet another schematic diagram illustrating a front projection of another device for monitoring a physiological signal according to some embodiments of the present disclosure.

Merely by way of example, as shown in FIG. 3Q, projected long edges 321-1 and 322-1 of the first electrode element 321 and the second electrode element 322, respectively, are contained within a projected long edge 350-1 of the first portion 350 of the second conductive yarns 312 (a side of the projected region of the first portion 350 that is parallel to the length direction of the strap 310), such that the conductive region corresponding to the first portion 350 of the second conductive yarns 312 covers the first electrode element 321 and the second electrode element 322 in the length direction of the strap 310.

In some embodiments, the conductive region formed by the first portion 350 of the second conductive yarns 312 covers the first electrode element and the second electrode element in the width direction of the strap.

FIG. 3R is yet another schematic diagram illustrating a side projection of another device for monitoring a physiological signal according to some embodiments of the present disclosure. The left and right diagrams in FIG. 3R are schematic side projection views observed from two opposite sides of the device 300 for monitoring a physiological signal.

Merely by way of example, as shown in FIG. 3R, the projected wide edges 321-2 and 322-2 of the first electrode element 321 and the second electrode element 322, respectively, are contained within a projected wide edge 350-2 of the first portion 350 of the second conductive yarns 312 (a side of the projected region of the first portion 350 that is parallel to the width direction of the strap 310), such that the conductive region covers the first electrode element 321 and the second electrode element 322 in the width direction of the strap 310.

In some embodiments of the present disclosure, by arranging the conductive region to cover the two electrode elements in the width direction, the conductive region may disperse static electricity near the outer surface directly facing the two electrode elements, thereby minimizing or eliminating the influence of the static electricity on the electrodes.

In some embodiments of the present disclosure, by electrically connecting two conductive regions to form one conductive region, the capability of the conductive region to disperse static electricity can be further enhanced. Moreover, by arranging the conductive region to cover the two electrode elements in the length direction, the influence of the static electricity on the electrodes can be minimized or eliminated to the greatest extent.

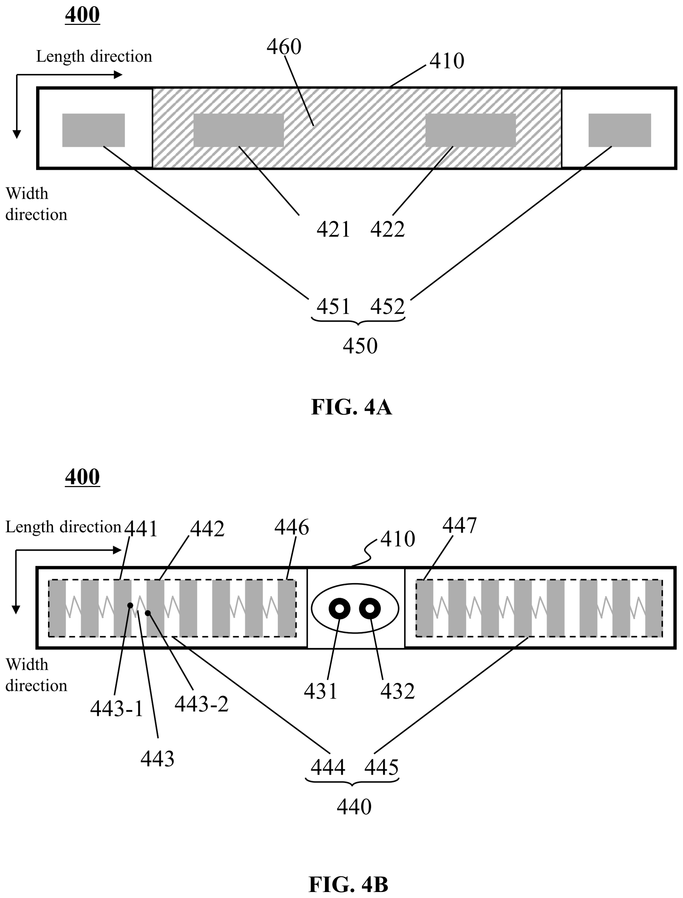

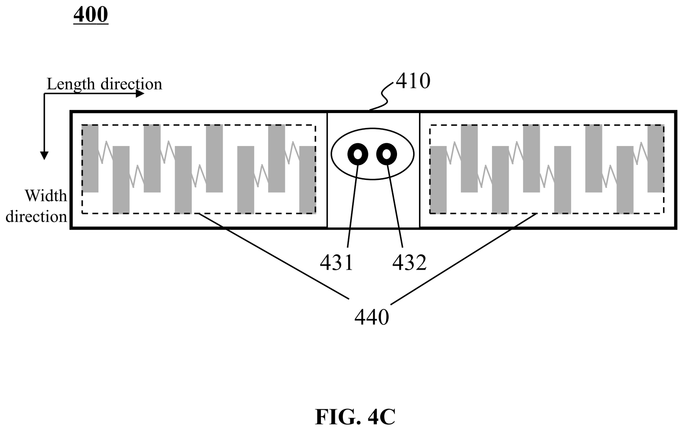

FIG. 4A is a structural diagram illustrating an inner surface of yet another device for monitoring a physiological signal according to some embodiments of the present disclosure; FIG. 4B is a structural diagram illustrating an outer surface of yet another device for monitoring a physiological signal according to some embodiments of the present disclosure; and FIG. 4C is another structural diagram illustrating an outer surface of yet another device for monitoring a physiological signal according to some embodiments of the present disclosure.

In some embodiments, as shown in FIGs. 4A and 4B, the device 400 for monitoring a physiological signal may include a strap 410, two electrode elements (including a third electrode element 421 and a fourth electrode element 422), a plurality of electrostatic protection sheets 440, one or more grounding electrodes 450, and a waterproof layer 460.

Similar to the strap 210 or the strap 310, the strap 410 serves as a base structure of the device 400 for monitoring a physiological signal and is configured to be worn on a body of a user (e.g., the monitored target 140), such that the two electrode elements may fit closely against the body of the user. For example, two ends of the strap 410 may be provided with buckles. When the two buckles are fastened together, the strap 410 may encircle and fit around a wearing portion of the monitored target 140 (e.g., encircle and fit around the waist of the user).

Similar to the strap 310, the material of the strap 410 may include plant fibers (e.g., cotton fibers, hemp fibers, etc.), animal fibers (e.g., wool, etc.), synthetic fibers (e.g., acrylic fibers, polyester fibers, etc.), etc. For example, the strap 410 may be woven from cotton fibers.

In some embodiments, the strap 410 includes at least an elastic yarn (e.g., a polyester yarn, a nylon yarn, etc.). The yarns used for weaving the strap 410 include at least the elastic yarn. For example, the strap 410 may be woven from the polyester yarn.

In some embodiments, the strap 410 includes at least the elastic yarn and the insulating yarn (e.g., a cotton yarn, a spandex yarn, etc.), that is, the yarns used for weaving the strap 410 include at least the elastic yarn and the insulating yarn. For example, the strap 410 may be woven by mixed weaving of the polyester yarn and the cotton yarn.

The two electrode elements (the third electrode element 421 and the fourth electrode element 422) are arranged at intervals in the length direction of the strap 410, and each of the two electrode elements includes an electrode configured to contact the body of the user to collect a physiological signal (e.g., an electrocardiogram signal).

Merely by way of example, as shown in FIG. 4A, the two electrode elements include the third electrode element 421 and the fourth electrode element 422. The third electrode element 421 and the fourth electrode element 422 are arranged at intervals in the length direction of the strap 410, and both electrode elements are disposed on the inner surface of the strap 410. Each of the third electrode element 421 and the fourth electrode element 422 includes an electrode (not shown in the figure), and the electrode is configured to receive an electrical signal from the body of the user. The inner surface of the strap 410 refers to a surface of the strap 410 that contacts the body of the user (i.e., a surface facing the skin of the human body), and the outer surface refers to another surface opposite to the inner surface of the strap 410 (i.e., a surface facing away from the skin of the human body).

In some embodiments, the device for monitoring a physiological signal may further be provided with two connection ports on the outer surface of the strap, and each of the two connection ports may be electrically connected to a corresponding electrode of the two electrode elements. The two connection ports may be connected to a transmitter (e.g., connected in a snap-fit manner), and the transmitter may transmit electrical signals (e.g., electrocardiogram potentials) collected by a plurality of electrodes to the processing device. The processing device may determine the physiological signal (e.g., the electrocardiogram signal) and/or physiological data (e.g., an electrocardiogram) of a monitored target (e.g., the monitored target 140) based on the electrical signals.

Merely by way of example, as shown in FIG. 4B, the device 400 for monitoring a physiological signal may further be provided with a connection port 431 and a connection port 432 on an outer surface of the strap 410. The two electrode elements, including the third electrode element 421 and the fourth electrode element 422, may be electrically connected to the connection port 431 and the connection port 432, respectively. The connection port 431 and the connection port 432 may be connected to a transmitter (not shown in the figure) (e.g., connected in a snap-fit manner), and the transmitter may transmit electrical signals (e.g., electrocardiogram potentials) collected by the third electrode element 421 and the fourth electrode element 422 to the processing device, so that the processing device may determine the physiological signals (e.g., the electrocardiogram signals) and/or physiological data (e.g., electrocardiograms) of a monitored target (e.g., the monitored target 140) based on the electrical signals. The connection port 431 and the connection port 432 may also be directly connected to the processing device (not shown in the figure) (e.g., detachably connected by the magnetic attachment). The processing device may directly obtain electrical signals collected by the third electrode element 421 and the fourth electrode element 422 through the connection port 431 and the connection port 432, and determine the physiological signals (e.g., the electrocardiogram signals) and/or physiological data (e.g., electrocardiograms) of a monitored target (e.g., the monitored target 140) based on the electrical signals.

The plurality of electrostatic protection sheets may be disposed on the outer surface of the strap 410, or between the inner surface and the outer surface of the strap 410, such that the two electrode elements (e.g., the third electrode element 421 and the fourth electrode element 422) are located between the electrostatic protection sheets and the body of the user (e.g., the monitored target 140). Merely by way of example, as shown in FIG. 4B, the plurality of electrostatic protection sheets 440 of the device 400 for monitoring a physiological signal are disposed on the outer surface of the strap 410 and cover the two electrode elements in the thickness direction of the strap 410, such that the two electrode elements are located between the plurality of electrostatic protection sheets 440 and the body of the user.

The electrostatic protection sheet refers to a conductive sheet used for receiving static electric charges on the outer surface of the device for monitoring a physiological signal (e.g., static electric charges generated by friction between the human body and clothing worn by the user). The material of the electrostatic protection sheet may be a conductive material with relatively low hardness, such as conductive rubber, conductive silicone, etc.

In some embodiments, the plurality of electrostatic protection sheets are arranged side by side in the length direction of the strap.

In some embodiments, as shown in FIG. 4B, the plurality of electrostatic protection sheets 440 are arranged side by side in the length direction of the strap 410. The parallel arrangement refers to a configuration in which reference points of each of the plurality of electrostatic protection sheets 440 are located on the same reference line, where the reference point of each electrostatic protection sheet may be preset (e.g., preset as a geometric center point of the electrostatic protection sheet). The reference line refers to a straight line in the strap 410 that is parallel to the length direction of the strap 410 (e.g., a center line in the strap 410 that is parallel to the length direction of the strap 410). Each electrostatic protection sheet of the plurality of electrostatic protection sheets 440 may have a preselected shape (e.g., as shown in FIG. 4B, each electrostatic protection sheet is rectangular). The length of each electrostatic protection sheet of the plurality of electrostatic protection sheets 440 in the length direction of the strap 410 may be the same or different, and the length of each electrostatic protection sheet of the plurality of electrostatic protection sheets 440 in the width direction of the strap 410 may also be the same or different.

In some embodiments, as shown in FIG. 4C, the plurality of electrostatic protection sheets 440 are arranged in a staggered manner in the length direction of the strap 410. Staggered arrangement refers to a configuration in which reference points of at least a portion of the plurality of electrostatic protection sheets 440 are not located on the same reference line.

In some embodiments, at least a portion of the plurality of electrostatic protection sheets are electrically connected to each other through conductive wires.

Merely by way of example, as shown in FIG. 4B, an electrostatic protection sheet 441 and an electrostatic protection sheet 442 among the plurality of electrostatic protection sheets 440 are connected to each other through a conductive wire 443, so that electrical conduction is achieved between the electrostatic protection sheet 441 and the electrostatic protection sheet 442.

In some embodiments of the present disclosure, at least a portion of the plurality of electrostatic protection sheets are electrically connected to each other through conductive wires, enabling the plurality of electrostatic protection sheets to form a large conductive region, which facilitates dispersing static electricity near the outer surface opposite to the signal electrode elements, thereby minimizing or eliminating the influence of the static electricity on the electrodes.

In some embodiments, the distance between connection points of two adjacent electrostatic protection sheets with a conductive wire is less than the natural length of the conductive wire.

Merely by way of example, as shown in FIG. 4B, a distance between connection points 443-1 and 443-2 of adjacent electrostatic protection sheets 441 and 442, with the conductive wire 443 is less than the natural length of the conductive wire 443.

To achieve the distance between connection points of two adjacent electrostatic protection sheets with the conductive wire less than the natural length of the conductive wire, a shape of the conductive wire between the two adjacent electrostatic protection sheets may be wavy, zigzag, or other shapes, without limitation.

In some embodiments of the present disclosure, by setting a distance between connection points of two adjacent electrostatic protection sheets with a conductive wire to be less than a natural length of the conductive wire, a stretchable length may be reserved for the conductive wire, thereby improving the extensibility of the conductive wire and further enhancing the extensibility of the device for monitoring a physiological signal.

In some embodiments, the conductive wire 443 is an elastic conductive wire that may elastically extend and contract in an axial direction of the conductive wire (parallel to the length direction of the strap 410 in FIG. 4B). For example, when the strap 410 is stretched, the conductive wire 443 correspondingly extends in the axial direction; when the strap 410 rebounds from a stretched state, the conductive wire 443 correspondingly shortens in the axial direction. The material of the conductive wire 443 may be a mixed wire formed by weaving a metal yarn (e.g., a silver yarn) and an elastic yarn (e.g., a rubber yarn) together, etc.

In some embodiments of the present disclosure, by configuring the conductive wire as the stretchable elastic conductive wire, the overall elasticity of the strap can be improved.

In some embodiments, the two electrode elements include the third electrode element and the fourth electrode element arranged at intervals in the length direction of the strap, and a plurality of electrostatic protection sheets are sequentially electrically connected through conductive wires (such that the plurality of electrostatic protection sheets are electrically connected to each other). The plurality of electrostatic protection sheets form the conductive region, and the conductive region covers the third electrode element and the fourth electrode element in the length direction of the strap.

Merely by way of example, as shown in FIGs. 4A and 4B, a plurality of signal electrodes 420 include the third electrode element 421 and the fourth electrode element 422 that are arranged at intervals in the length direction of the strap 410. As shown in FIG. 4B, at least a portion of adjacent electrostatic protection sheets 440 among the plurality of electrostatic protection sheets 440 are electrically connected through conductive wires (e.g., the conductive wire 443), and the plurality of electrostatic protection sheets 440 form an integrated conductive region (a region where the plurality of electrostatic protection sheets 440 are located in FIG. 4B). In order to enable the plurality of electrostatic protection sheets 440 to form an integrated conductive region, a first portion of the plurality of electrostatic protection sheets 440 (e.g., a plurality of electrostatic protection sheets on the left side) and a second portion of the plurality of electrostatic protection sheets 440 (e.g., a plurality of electrostatic protection sheets on the right side) are electrically connected, i.e., a last electrostatic protection sheet 446 of the first portion is electrically connected to a first electrostatic protection sheet 447 of the second portion (not shown in the figure).

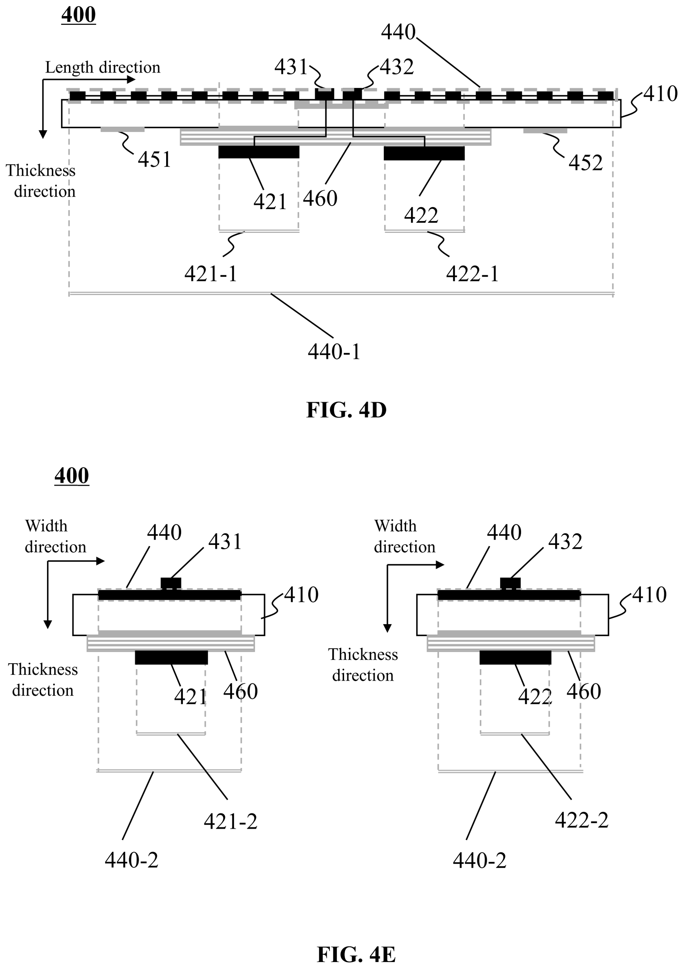

FIG. 4D is a schematic diagram illustrating a front projection of yet another device for monitoring a physiological signal according to some embodiments of the present disclosure.

Merely by way of example, as shown in FIG. 4D, a projection long side 421-1 of the third electrode element 421 (a side of the projected region of the third electrode element 421 that is parallel to the length direction of the strap 410) and a projection long side 422-1 of the fourth electrode element 422 (a side of the projected region of the fourth electrode element 422 that is parallel to the length direction of the strap 410) are both contained within a projection long side 440-1 of the conductive region formed by the plurality of electrostatic protection sheets 440 (a side of the projected region of the conductive region formed by the plurality of electrostatic protection sheets 440 that is parallel to the length direction of the strap 410), such that the conductive region formed by the plurality of electrostatic protection sheets 440 covers the third electrode element 421 and the fourth electrode element 422 in the length direction of the strap 410.

In some embodiments of the present disclosure, since the static electricity that most affects the signal electrodes is the static electricity on and near the outer surface directly facing the signal electrodes, by configuring the conductive region to cover the two signal electrode elements, the conductive region may disperse the static electricity on and near the outer surface directly facing the signal electrode elements, thereby minimizing or eliminating the influence of the static electricity on the electrodes.

In some embodiments, the conductive region covers the third electrode element and the fourth electrode element in the width direction of the strap.

FIG. 4E is a schematic diagram illustrating a side projection of yet another device for monitoring a physiological signal according to some embodiments of the present disclosure. The left diagram and the right diagram in FIG. 4E are schematic side projection views observed from two opposite sides of the device 400 for monitoring a physiological signal.

Merely by way of example, as shown in FIG. 4E, a projected width edge 421-2 of the third electrode element 421 (a side of the projected region of the third electrode element 421 that is parallel to the width direction of the strap 410) and a projected width edge 422-2 of the fourth electrode element 422 (a side of the projected region of the fourth electrode element 422 that is parallel to the width direction of the strap 410) are both contained within a projected width edge 440-2 of the conductive region formed by the plurality of electrostatic protection sheets 440 (a side of the projected region of the conductive region formed by the plurality of electrostatic protection sheets 440 that is parallel to the width direction of the strap 410), such that the conductive region covers the third electrode element 421 and the fourth electrode element in the width direction of the strap 410.

In some embodiments of the present disclosure, by disposing the conductive region to cover two signal electrode elements in the width direction, the conductive region may disperse static electricity near the outer surface directly facing the two signal electrode elements, thereby minimizing or eliminating the influence of the static electricity on the electrodes to the greatest extent.

In some embodiments, the two electrode elements include a third electrode element and a fourth electrode element that are arranged at intervals in the length direction of the strap. A portion of the plurality of electrostatic protection sheets is electrically connected through conductive wires to form a first conductive region, the first conductive region is disposed on an outer side of the third electrode element, another portion of the plurality of electrostatic protection sheets is electrically connected through conductive wires to form a second conductive region, the second conductive region is disposed on an outer side of the fourth electrode element, and the first conductive region and the second conductive region are insulated from each other.

Merely by way of example, as shown in FIG. 4B, at least a portion of adjacent electrostatic protection sheets in the first portion (e.g., the plurality of electrostatic protection sheets on the left side) of the plurality of electrostatic protection sheets 440 are electrically connected in sequence through conductive wires (e.g., the conductive wire 443) to form a first conductive region 444. The first conductive region 444 is disposed on the outer side of the third electrode element 421. At least a portion of adjacent electrostatic protection sheets in the second portion (e.g., the plurality of electrostatic protection sheets on the right side) of the plurality of electrostatic protection sheets 440 are electrically connected in sequence through conductive wires to form a second conductive region 445. The second conductive region 445 is disposed on the outer side of the fourth electrode element 422. The first conductive region 444 and the second conductive region 445 are insulated from each other, i.e., there is no electrical conduction between the last electrostatic protection sheet 446 of the first portion and the first electrostatic protection sheet 447 of the second portion.

In some embodiments of the present disclosure, by disposing different conductive regions to cover corresponding electrode elements, the different conductive regions may disperse static electricity near the outer surface directly facing the electrode elements, thereby minimizing or eliminating the influence of the static electricity on the electrodes to the greatest extent.

In some embodiments, the first conductive region covers the third electrode element in the length direction of the strap, and the second conductive region covers the fourth electrode element in the length direction of the strap.

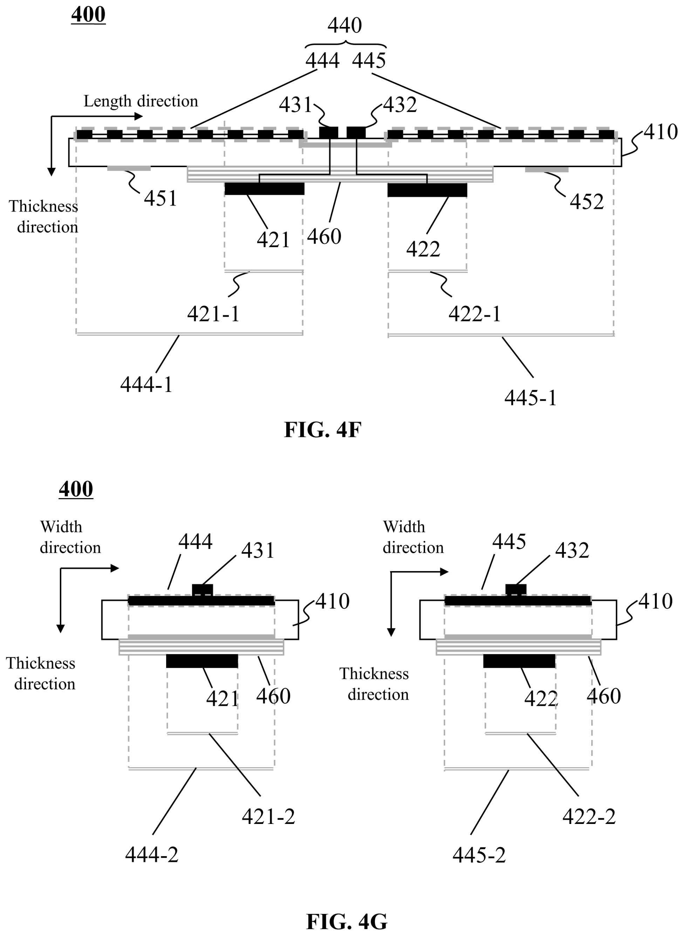

FIG. 4F is another schematic diagram illustrating a front projection of yet another device for monitoring a physiological signal according to some embodiments of the present disclosure.

Merely by way of example, as shown in FIG. 4F, the projected long edge 421-1 of the third electrode element 421 is contained within a projected long edge 444-1 of the first conductive region 444, which is a side of a projected region of the first conductive region 444 that is parallel to the length direction of the strap 410. The projected long edge 422-1 of the fourth electrode element 422 is contained within a projected long edge 445-1 of the second conductive region 445, which is a side of a projected region of the second conductive region 445 that is parallel to the length direction of the strap 410. In this way, the first conductive region 444 is arranged to cover the third electrode element 421 in the length direction of the strap 410, and the second conductive region 445 is arranged to cover the fourth electrode element 422 in the length direction of the strap 410.

In some embodiments of the present disclosure, by arranging different conductive regions to cover corresponding electrode elements in the length direction of the strap, different conductive regions are enabled to disperse static electricity near the outer surface directly facing the electrode elements, thereby minimizing or eliminating the influence of the static electricity on the electrodes.

In some embodiments, the first conductive region is arranged to cover the third electrode element in the width direction of the strap, and the second conductive region is arranged to cover the fourth electrode element in the width direction of the strap.

FIG. 4G is another schematic diagram illustrating a side projection of yet another device for monitoring a physiological signal according to some embodiments of the present disclosure. The left and right diagrams in FIG. 4G are schematic side projection views observed from two opposite sides of the device 400 for monitoring a physiological signal.

Merely by way of example, as shown in FIG. 4G, the projected width edge 421-2 of the third electrode element 421 is contained within a projected width edge 444-2 of the first conductive region 444, which is a side of the projected region of the first conductive region 444 that is parallel to the width direction of the strap 410. The projected width edge 422-2 of the fourth electrode element 422 is contained within a projected width edge 445-2 of the second conductive region 445, which is a side of the projected region of the second conductive region 445 that is parallel to the width direction of the strap 410. The first conductive region 444 is arranged to cover the third electrode element 421 in the width direction of the strap 410, and the second conductive region 445 is arranged to cover the fourth electrode element 422 in the width direction of the strap 410.

In some embodiments of the present disclosure, by arranging different conductive regions to cover corresponding electrode elements in the width direction of the strap, different conductive regions are enabled to disperse static electricity near the outer surface directly facing the electrode elements, thereby minimizing or eliminating the influence of the static electricity on the electrodes.

The grounding electrode is located on the inner surface of the strap and is configured to electrically connect the electrostatic protection sheets to the body of the user to implement the electrostatic shielding for the two electrode elements. The material of the grounding electrode may be a conductive material with low hardness, such as conductive rubber or conductive silicone. Merely by way of example, as shown in FIGs. 4A and 4B, the one or more grounding electrodes 450 of the device 400 for monitoring a physiological signal may include a grounding electrode 451 and a grounding electrode 452, which are disposed on the inner surface of the strap 410. When the user (e.g., the monitored target 140) wears the device 400 for monitoring a physiological signal, the one or more grounding electrodes 450 are in contact with the skin of the user. The one or more grounding electrodes 450 are electrically connected to the plurality of electrostatic protection sheets 440 (not shown in the figure) to receive static charges on the outer surface collected by the plurality of electrostatic protection sheets 440 and guide the static charges into the body to implement the electrostatic shielding.

Similar to the waterproof layer 330, the waterproof layer 460 refers to a structure in the device 400 for monitoring a physiological signal that provides waterproof and insulating functions. The two electrode elements (the third electrode element 421 and the fourth electrode element 422) are both connected to the strap 410 through the waterproof layer 460. Specifically, in the thickness direction of the strap 410, the waterproof layer 460 is located between the strap 410 and the electrode elements. When the strap 410 is soaked with a liquid (e.g., sweat or water), the waterproof layer 460 prevents the liquid from spreading to the electrode elements, thereby achieving waterproofing of the electrodes.

In some embodiments, the waterproof layer 460 includes a waterproof insulating yarn (e.g., an acrylic yarn or a spandex yarn). In other words, the yarn used for weaving the waterproof layer 460 includes the waterproof insulating yarn. For example, the waterproof layer 460 may be woven from the acrylic yarn.

n some embodiments, the waterproof layer and the strap are woven in a spliced manner, and the waterproof layer extends through the inner surface and the outer surface of the strap in the thickness direction of the strap.

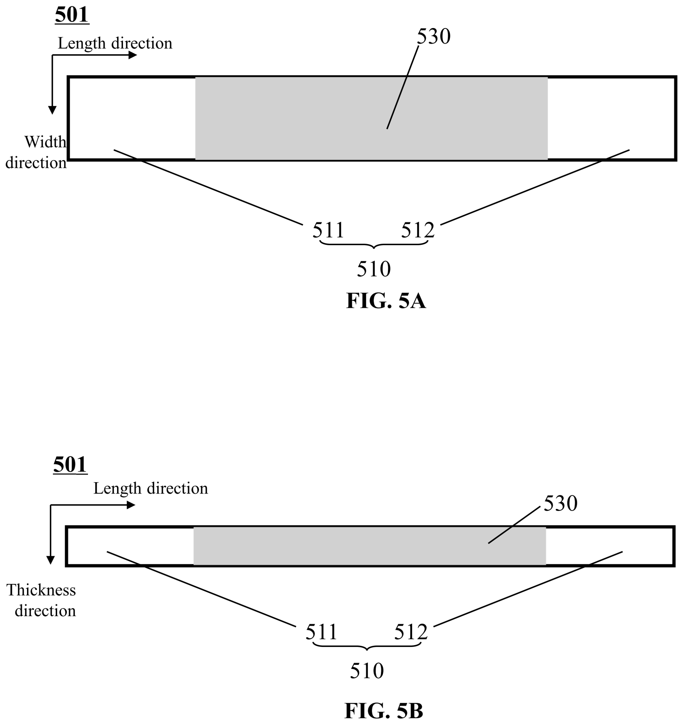

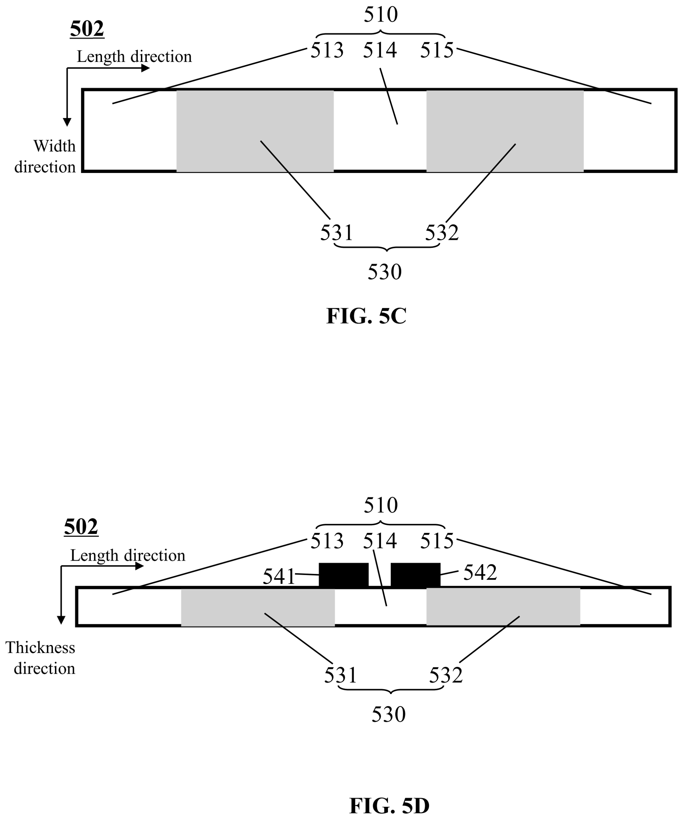

FIG. 5A is a structural diagram illustrating a surface structure of a substrate of yet another device for monitoring a physiological signal according to some embodiments of the present disclosure; FIG. 5B is a front view of a substrate structure of yet another device for monitoring a physiological signal according to some embodiments of the present disclosure; FIG. 5C is a structural diagram illustrating a surface structure of another substrate of yet another device for monitoring a physiological signal according to some embodiments of the present disclosure; and FIG. 5D is a front view of another substrate structure of yet another device for monitoring a physiological signal according to some embodiments of the present disclosure.

As shown in FIGs. 5A-5D, the base structure (base structures 501 and 502) of the device for monitoring a physiological signal may include a strap 510 and a waterproof layer 530. The base structure of the device for monitoring a physiological signal is configured to be worn on the body of the user (e.g., the monitored target 140), for example, on the chest, back, or waist of the user, so that the two electrode elements may be in contact with the body of the user. For example, both ends of the base structure may be provided with buckles. When the buckles at both ends are engaged, the base structure may encircle and fit closely against the body of the user (e.g., around the waist of the user).

In some embodiments, a material of the strap 510 may include plant fibers (e.g., a cotton fiber or a linen fiber), animal fibers (e.g., wool), or synthetic fibers (e.g., an acrylic fiber or a polyester fiber), etc. For example, the strap 510 may be woven from the cotton fiber.

In some embodiments, the strap 510 includes at least an elastic yarn (e.g., a polyester yarn or a nylon yarn), i.e., the yarns used for weaving the strap 510 include at least the elastic yarn. For example, the strap 510 may be woven from the polyester yarn.

In some embodiments, the strap 510 includes at least an elastic yarn and an insulating yarn (e.g., the cotton yarn or the spandex yarn), that is, the yarns used for weaving the strap 510 include at least the elastic yarn and the insulating yarn. For example, the strap 510 may be woven from the polyester yarn and the cotton yarn in a mixed weaving manner.

In some embodiments, the strap 510 may be formed by mixed weaving of the elastic yarn and the insulating yarn.

In some embodiments, as shown in FIGs. 5A and 5B, the strap 510 may include a first strap portion 511 and a second strap portion 512. The first strap portion 511 and the second strap portion 512 may be formed by mixed weaving of the elastic yarn and the insulating yarn, respectively.

In some embodiments, as shown in FIGs. 5C and 5D, the strap 510 may include a third strap portion 513, a fourth strap portion 514, and a fifth strap portion 515. The third strap portion 513, the fourth strap portion 514, and the fifth strap portion 515 may be formed by mixed weaving of the elastic yarn and the insulating yarn.

When the strap 510 is soaked with a liquid (e.g., sweat or water), the waterproof layer 530 may prevent the liquid from spreading to the electrode elements, thereby achieving waterproofing for the electrodes.

In some embodiments, the waterproof layer 530 includes the waterproof insulating yarn (e.g., the acrylic yarn or the spandex yarn), that is, the yarns used for weaving the waterproof layer 530 include the waterproof insulating yarn. For example, the waterproof layer 530 may be woven from the acrylic yarn.

In some embodiments, as shown in FIGs. 5C and 5D, the waterproof layer 530 may include a first waterproof portion 531 and a second waterproof portion 532. The first waterproof portion 531 and the second waterproof portion 532 may be woven from the waterproof insulating yarns, respectively.

In some embodiments, to obtain the base structure 501 as shown in FIGs. 5A and 5B, the weaving process of the base structure 501 may include: mixing and weaving the elastic yarn and the insulating yarn to form the first strap portion 511 and the second strap portion 512; weaving the waterproof insulating yarn to form the waterproof layer 530; and splicing the first strap portion 511, the second strap portion 512, and the waterproof layer 530 according to the positional relationship shown in FIGs. 5A and 5B (where the first strap portion 511 and the second strap portion 512 are located on two sides of the waterproof layer 530), thereby obtaining the base structure 501. The splicing method described above may include: using yarns (e.g., the insulating yarn or the waterproof insulating yarn) to sew and splice the adjacent side edges between the first strap portion 511 and the waterproof layer 530, and between the waterproof layer 530 and the second strap portion 512; or using an adhesive (e.g., latex) to bond and splice the adjacent side edges between the first strap portion 511 and the waterproof layer 530, and between the waterproof layer 530 and the second strap portion 512.

In some embodiments, to obtain the base structure 501 as shown in FIGs. 5A and 5B, the strap 510 and the waterproof layer 530 included in the base structure 501 may be integrally woven. The above integrated weaving process may be as follows: based on the yarns contained in the strap 510 (including the first strap portion 511 and the second strap portion 512) and the waterproof layer 530 (where the strap 510 includes mixed yarns of the elastic yarn and the insulating yarn, and the waterproof layer 530 includes the waterproof insulating yarn), the strap 510 and the waterproof layer 530 are continuously woven according to the positional relationship among the first strap portion 511, the second strap portion 512, and the waterproof layer 530 shown in FIGs. 5A and 5B, to obtain a formed base structure 501. During the weaving process, when transitioning among the first strap portion 511, the second strap portion 512, and the waterproof layer 530, a transition weaving technique (e.g., tuck knitting) may be employed.

In some embodiments, to obtain the base structure 502 as shown in FIGs. 5C and 5D, the weaving process of the base structure 502 may include: mixing and weaving the elastic yarn and the insulating yarn to form the third strap portion 513, the fourth strap portion 514, and the fifth strap portion 515; weaving the waterproof insulating yarns separately to form the first waterproof portion 531 and the second waterproof portion 532; and splicing the third strap portion 513, the first waterproof portion 531, the fourth strap portion 514, the second waterproof portion 532, and the fifth strap portion 515 in sequence according to the positional relationship shown in FIGs. 5C and 5D (i.e., sequentially arranged in the length direction of the base structure 502 are the third strap portion 513, the first waterproof portion 531, the fourth strap portion 514, the second waterproof portion 532, and the fifth strap portion 515), thereby obtaining the base structure 502. The splicing method described above may include: using yarns (e.g., the insulating yarn or the waterproof insulating yarn) to sew and splice the adjacent side edges between the third strap portion 513 and the first waterproof portion 531, between the first waterproof portion 531 and the fourth strap portion 514, between the fourth strap portion 514 and the second waterproof portion 532, and between the second waterproof portion 532 and the fifth strap portion 515; or using an adhesive (e.g., latex) to bond and splice the adjacent side edges between the third strap portion 513 and the first waterproof portion 531, between the first waterproof portion 531 and the fourth strap portion 514, between the fourth strap portion 514 and the second waterproof portion 532, and between the second waterproof portion 532 and the fifth strap portion 515.

In some embodiments, to obtain the base structure 502 as shown in FIGs. 5C and 5D, the strap 510 and the waterproof layer 530 included in the base structure 502 may be integrally woven. The above integrated weaving process may be as follows: based on the yarns contained in the strap 510 (including the third strap portion 513, the fourth strap portion 514, and the fifth strap portion 515) and the waterproof layer 530 (including the first waterproof portion 531 and the second waterproof portion 532), where the strap 510 includes a mixed yarn of the elastic yarn and the insulating yarn, and the waterproof layer 530 includes the waterproof insulating yarn, the strap 510 and the waterproof layer 530 are continuously woven according to the positional relationship among the third strap portion 513, the fourth strap portion 514, the fifth strap portion 515, the first waterproof portion 531, and the second waterproof portion 532 as shown in FIGs. 5C and 5D, to obtain the formed base structure 502. During the weaving process, when transitioning among the third strap portion 513, the fourth strap portion 514, the fifth strap portion 515, the first waterproof portion 531, and the second waterproof portion 532, a transition weaving technique (e.g., tuck knitting) may be employed.

In some embodiments, by weaving the waterproof layer and the strap in a spliced manner, the manufacturing process of the base structure of the device for monitoring a physiological signal can be simplified, the process duration can be shortened, the overall thickness of the device for monitoring a physiological signal can be reduced, and the wearing comfort of the user can be improved.

In some embodiments, the device for monitoring a physiological signal further includes electrode elements, and the electrode elements are woven on an inner side of the waterproof layer.

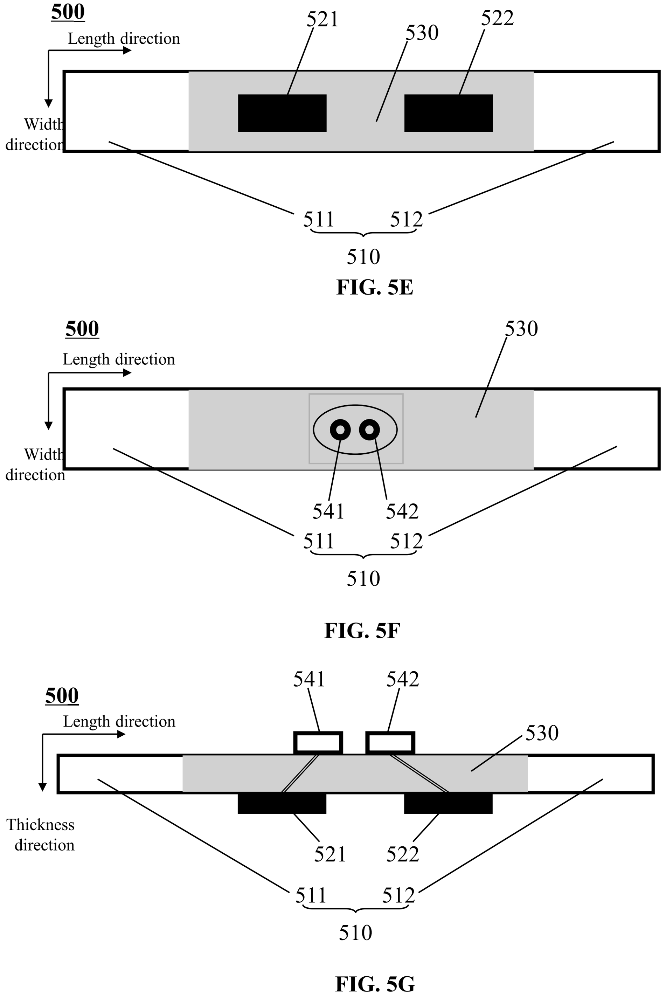

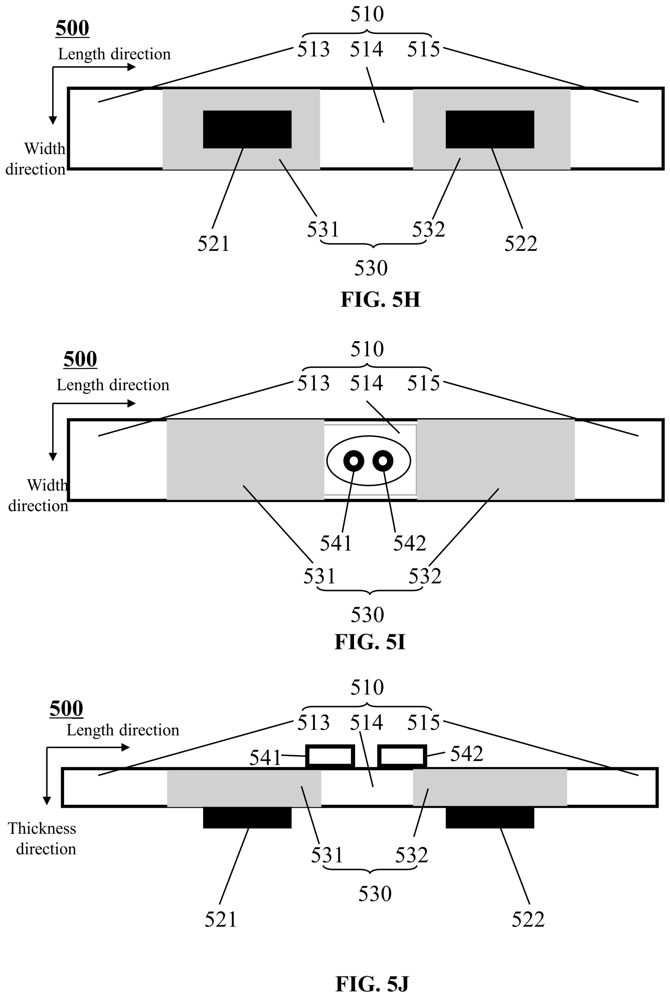

FIG. 5E is a structural diagram illustrating an inner surface of yet another device for monitoring a physiological signal according to some embodiments of the present disclosure; FIG. 5F is a structural diagram illustrating an outer surface of yet another device for monitoring a physiological signal according to some embodiments of the present disclosure; FIG. 5G is a front view of yet another device for monitoring a physiological signal according to some embodiments of the present disclosure; FIG. 5H is another structural diagram illustrating an inner surface of yet another device for monitoring a physiological signal according to some embodiments of the present disclosure; FIG. 5I is another structural diagram illustrating an outer surface of yet another device for monitoring a physiological signal according to some embodiments of the present disclosure; and FIG. 5J is another front view of yet another device for monitoring a physiological signal according to some embodiments of the present disclosure.

As shown in FIGs. 5E to 5G, based on the base structure 501 shown in FIGs. 5A and 5B, two electrode elements (including a fifth electrode element 521 and a sixth electrode element 522) may be woven at intervals on an inner side (an inner surface) of the waterproof layer 530, thereby obtaining the device 500 for monitoring a physiological signal.

As shown in FIGs. 5H to 5J, based on the base structure 502 shown in FIGs. 5C and 5D, the two electrode elements (including the electrode element 521 and the electrode element 522) may be respectively woven on inner sides (inner surfaces) of the first waterproof portion 531 and the second waterproof portion 532, thereby obtaining the device 500 for monitoring a physiological signal.

Each of the two electrode elements (the fifth electrode element 521 and the sixth electrode element 522) includes an electrode (not shown in the figures) as described in other portions of the present disclosure, and the electrode is configured to receive electrical signals from the body of the user.

In some embodiments of the present disclosure, based on the device 500 for monitoring a physiological signal shown in FIGs. 5E to 5J, in which the electrode elements are woven on inner surface of the waterproof layer, the waterproof layer may prevent liquid in the strap from spreading to the electrode elements, thereby achieving waterproofing of the electrodes.

In some embodiments, the electrode and the waterproof layer are woven in a spliced manner, and the waterproof layer is located between the strap and the two electrode elements.

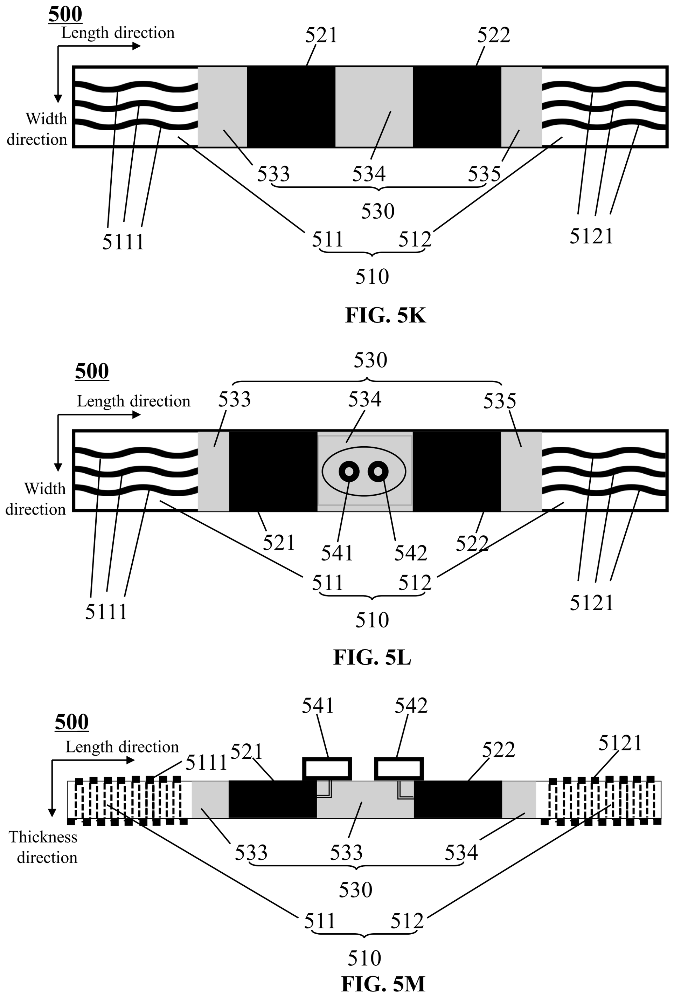

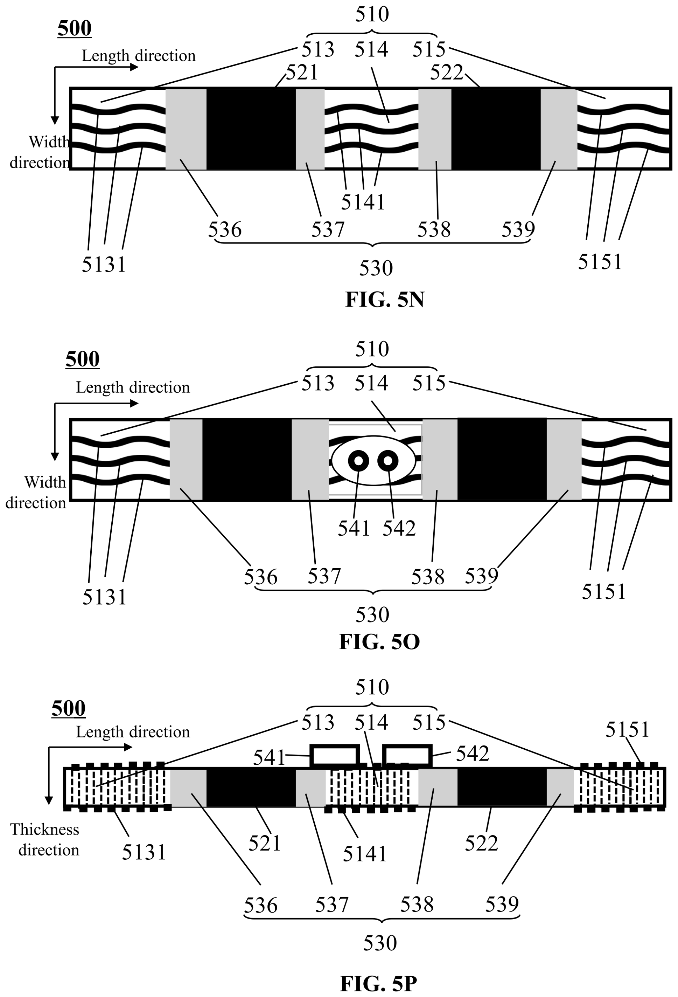

FIG. 5K is a structural diagram illustrating an inner surface of yet another device for monitoring a physiological signal according to some embodiments of the present disclosure; FIG. 5L is a structural diagram illustrating an outer surface of yet another device for monitoring a physiological signal according to some embodiments of the present disclosure; FIG. 5M is a front view of yet another device for monitoring a physiological signal according to some embodiments of the present disclosure; FIG. 5N is another structural diagram illustrating an inner surface of yet another device for monitoring a physiological signal according to some embodiments of the present disclosure; FIG. 5O is another structural diagram illustrating an outer surface of yet another device for monitoring a physiological signal according to some embodiments of the present disclosure; and FIG. 5P is another front view of yet another device for monitoring a physiological signal according to some embodiments of the present disclosure.

As shown in FIGs. 5K to 5M, the waterproof layer 530 may include a third waterproof portion 533, a fourth waterproof portion 534, and a fifth waterproof portion 535. The third waterproof portion 533, the fourth waterproof portion 534, and the fifth waterproof portion 535 may each be woven from the waterproof insulating yarns.

In some embodiments, to obtain the device 500 for monitoring a physiological signal as shown in FIGs. 5K to 5M, the weaving process of the device 500 for monitoring a physiological signal may include: mixing and weaving the elastic yarn and the insulating yarn to form the first strap portion 511 and the second strap portion 512; weaving the waterproof insulating yarn to form the third waterproof portion 533, the fourth waterproof portion 534, and the fifth waterproof portion 535; weaving the first conductive yarn to form the fifth electrode element 521 and the sixth electrode element 522; and splicing the first strap portion 511, the third waterproof portion 533, the fifth electrode element 521, the fourth waterproof portion 534, the sixth electrode element 522, the fifth waterproof portion 535, and the second strap portion 512 in sequence according to the positional relationship shown in FIGs. 5K to 5M (i.e., sequentially arranged in the length direction of the device 500 for monitoring a physiological signal are the first strap portion 511, the third waterproof portion 533, the fifth electrode element 521, the fourth waterproof portion 534, the sixth electrode element 522, the fifth waterproof portion 535, and the second strap portion 512), thereby obtaining the device 500 for monitoring a physiological signal. The splicing method may include: sewing adjacent side edges of the above portions (including adjacent side edges between the first strap portion 511 and the third waterproof portion 533, between the third waterproof portion 533 and the fifth electrode element 521, between the fifth electrode element 521 and the fourth waterproof portion 534, between the fourth waterproof portion 534 and the sixth electrode element 522, between the sixth electrode element 522 and the fifth waterproof portion 535, and between the fifth waterproof portion 535 and the second strap portion 512) using yarns (e.g., the insulating yarn or the waterproof insulating yarn), or bonding the adjacent side edges of the above portions using an adhesive (e.g., latex).

In some embodiments, to obtain the device 500 for monitoring a physiological signal as shown in FIGs. 5K to 5M, the strap 510, the waterproof layer 530, and the two electrode elements (including the fifth electrode element 521 and the sixth electrode element 522) included in the device 500 for monitoring a physiological signal may be formed by the integrated weaving. The integrated weaving process may be as follows: based on the yarns included in the strap 510 (including the first strap portion 511 and the second strap portion 512), the waterproof layer 530 (including the third waterproof portion 533, the fourth waterproof portion 534, and the fifth waterproof portion 535), and the electrode elements, where the strap 510 includes a mixed yarn of the elastic yarn and the insulating yarn, the waterproof layer 530 includes a waterproof insulating yarn, and the electrode elements include the first conductive yarn, the strap 510, the waterproof layer 530, and the two electrode elements are continuously woven according to the positional relationship shown in FIGs. 5K to 5M to obtain the formed device 500 for monitoring a physiological signal. During the weaving process, when transitioning among the first strap portion 511, the third waterproof portion 533, the fifth electrode element 521, the fourth waterproof portion 534, the sixth electrode element 522, the fifth waterproof portion 535, and the second strap portion 512, a transition weaving technique (e.g., tuck knitting) may be used.

As shown in FIGs. 5N to 5P, the waterproof layer 530 may include a sixth waterproof portion 536, a seventh waterproof portion 537, an eighth waterproof portion 538, and a ninth waterproof portion 539. The sixth waterproof portion 536, the seventh waterproof portion 537, the eighth waterproof portion 538, and the ninth waterproof portion 539 may be woven from the waterproof insulating yarns.

In some embodiments, to obtain the device 500 for monitoring a physiological signal as shown in FIGs. 5N to 5P, the weaving process of the device 500 for monitoring a physiological signal may include: mixing and weaving the elastic yarn and the insulating yarn to form the third strap portion 513, the fourth strap portion 514, and the fifth strap portion 515; weaving the waterproof insulating yarn to form the sixth waterproof portion 536, the seventh waterproof portion 537, the eighth waterproof portion 538, and the ninth waterproof portion 539; weaving the first conductive yarn to form the fifth electrode element 521 and the sixth electrode element 522; and splicing the third strap portion 513, the sixth waterproof portion 536, the fifth electrode element 521, the seventh waterproof portion 537, the fourth strap portion 514, the eighth waterproof portion 538, the sixth electrode element 522, the ninth waterproof portion 539, and the fifth strap portion 515 in sequence according to the positional relationship shown in FIGs. 5N to 5P (i.e., sequentially arranged in the length direction of the device 500 for monitoring a physiological signal are the third strap portion 513, the sixth waterproof portion 536, the fifth electrode element 521, the seventh waterproof portion 537, the fourth strap portion 514, the eighth waterproof portion 538, the sixth electrode element 522, the ninth waterproof portion 539, and the fifth strap portion 515), thereby obtaining the device 500 for monitoring a physiological signal. The splicing method may include: sewing the adjacent side edges of the above portions (including the adjacent side edges between the third strap portion 513 and the sixth waterproof portion 536, the sixth waterproof portion 536 and the fifth electrode element 521, the fifth electrode element 521 and the seventh waterproof portion 537, the seventh waterproof portion 537 and the fourth strap portion 514, the fourth strap portion 514 and the eighth waterproof portion 538, the eighth waterproof portion 538 and the sixth electrode element 522, the sixth electrode element 522 and the ninth waterproof portion 539, and the ninth waterproof portion 539 and the fifth strap portion 515) using yarns (e.g., the insulating yarn or the waterproof insulating yarn). Alternatively, the adjacent side edges of the above portions may be bonded together using an adhesive (e.g., latex).

In some embodiments, to obtain the device 500 for monitoring a physiological signal as shown in FIGs. 5N to 5P, the strap 510, the waterproof layer 530, and two electrode elements (including the fifth electrode element 521 and the sixth electrode element 522) included in the device 500 for monitoring a physiological signal may be integrally woven. The above integrated weaving process may be as follows: based on the yarns included in the strap 510 (including the third strap portion 513, the fourth strap portion 514, and the fifth strap portion 515), the waterproof layer 530 (including the sixth waterproof portion 536, the seventh waterproof portion 537, the eighth waterproof portion 538, and the ninth waterproof portion 539), and the electrode elements, where the strap 510 includes a mixed yarn of the elastic yarn and the insulating yarn, the waterproof layer 530 includes the waterproof insulating yarn, and the electrode elements include the first conductive yarn, the strap 510, the waterproof layer 530, and the two electrode elements are continuously woven according to the positional relationship shown in FIGs. 5N to 5P to obtain the formed device 500 for monitoring a physiological signal. During the weaving process, when transitioning among the third strap portion 513, the sixth waterproof portion 536, the fifth electrode element 521, the seventh waterproof portion 537, the fourth strap portion 514, the eighth waterproof portion 538, the sixth electrode element 522, the ninth waterproof portion 539, and the fifth strap portion 515, a transition weaving technique (e.g., tuck knitting) is required.

In some embodiments of the present disclosure, based on the structure of the device 500 for monitoring a physiological signal shown in FIGs. 5K to 5P (in which the waterproof layer is located between the strap and the electrode elements), the waterproof layer may prevent liquid in the strap from spreading to the electrode elements, thereby achieving waterproofing of the electrodes. In addition, the overall thickness of the device 500 for monitoring a physiological signal is reduced, and the wearing comfort of the user is improved.

In some embodiments, the strap includes a second conductive yarn. The second conductive yarn is interwoven through the inner surface and the outer surface of the strap, and the second conductive yarn implements electrostatic shielding for each of the two electrode elements.

As shown in FIGs. 5K-5M, a plurality of second conductive yarns 5111 are interwoven through the inner surface and the outer surface of the first strap portion 511, and a plurality of second conductive yarns 5121 are interwoven through the inner surface and the outer surface of the second strap portion 512 to implement the electrostatic shielding.

The outer portion of the plurality of second conductive yarns 5111 is located on the outer surface of the first strap portion 511, or between the inner surface and the outer surface of the first strap portion 511. The inner portion of the plurality of second conductive yarns 5111 (i.e., the portion of the plurality of second conductive yarns 5111 located on the inner surface of the first strap portion 511) extends to the inner surface of the first strap portion 511 and electrically connects the outer portion of the plurality of second conductive yarns 5111 to the body of the user. The outer portion of the plurality of second conductive yarns 5121 is located on the outer surface of the second strap portion 512, or between the inner surface and the outer surface of the second strap portion 512. The inner portion of the plurality of second conductive yarns 5121 (i.e., the portion of the plurality of second conductive yarns 5121 located on the inner surface of the second strap portion 512) extends to the inner surface of the second strap portion 512 and electrically connects the outer portion of the plurality of second conductive yarns 5121 to the body of the user. According to such arrangement, the electrostatic shielding for each of the electrode elements is implemented.

To implement the structure of the device 500 for monitoring a physiological signal described in the above embodiments, the weaving process of the first strap portion 511 and the second strap portion 512 may be as follows. First, the first strap portion 511 and the second strap portion 512 are woven and formed based on the elastic yarn and the insulating yarn. Subsequently, each strand of the plurality of second conductive yarns 5111 is interwoven through the inner surface and the outer surface of the first strap portion 511, and each strand of the plurality of second conductive yarns 5121 is interwoven through the inner surface and the outer surface of the second strap portion 512.

Taking the interweaving of the plurality of second conductive yarns 5111 as an example, a specific process of the interweaving may include: weaving the plurality of second conductive yarns 5111 in parallel. For example, each strand of the plurality of second conductive yarns 5111 is interwoven in the length direction of the first strap portion 511 with a certain spacing (either equal spacing or unequal spacing) in the width direction of the first strap portion 511, where a length of each strand of the second conductive yarn is sufficient to complete weaving from a head to a tail in the length direction of the first strap portion 511, thereby obtaining the woven plurality of second conductive yarns 5111. The interweaving manners of the plurality of second conductive yarns 5121 are similar.

Based on the structure of the device 500 for monitoring a physiological signal, as shown in FIGs. 5K-5M, when the outer surface of the first strap portion 511 or the outer surface of the second strap portion 512 carries static electricity, static charges may sequentially enter the human body via the outer portion and the inner portion of the plurality of second conductive yarns 5111 or the outer portion and the inner portion of the plurality of second conductive yarns 5121, such that the static charges cannot affect the fifth electrode element 521 and the sixth electrode element 522.

As shown in FIGs. 5N-5P, a plurality of second conductive yarns 5131 are interwoven through the inner surface and the outer surface of the third strap portion 513, a plurality of second conductive yarns 5141 are interwoven through the inner surface and the outer surface of the fourth strap portion 514, and a plurality of second conductive yarns 5151 are interwoven through the inner surface and the outer surface of the fifth strap portion 515 to implement the electrostatic shielding.

The outer portion of the plurality of second conductive yarns 5131 is located on the outer surface of the third strap portion 513, or between the inner surface and the outer surface of the third strap portion 513. The inner portion of the plurality of second conductive yarns 5131 (i.e., the portion of the plurality of second conductive yarns 5131 located on the inner surface of the third strap portion 513) extends to the inner surface of the third strap portion 513 and electrically connects the outer portion of the plurality of second conductive yarns 5131 to the body of the user. The outer portion of the plurality of second conductive yarns 5141 is located on the outer surface of the fourth strap portion 514, or between the inner surface and the outer surface of the fourth strap portion 514. The inner portion of the plurality of second conductive yarns 5141 (i.e., the portion of the plurality of second conductive yarns 5141 located on the inner surface of the fourth strap portion 514) extends to the inner surface of the fourth strap portion 514 and electrically connects the outer portion of the plurality of second conductive yarns 5141 to the body of the user. The outer portion of the plurality of second conductive yarns 5151 is located on the outer surface of the fifth strap portion 515, or between the inner surface and the outer surface of the fifth strap portion 515. The inner portion of the plurality of second conductive yarns 5151 (i.e., the portion of the plurality of second conductive yarns 5151 located on the inner surface of the fifth strap portion 515) extends to the inner surface of the fifth strap portion 515 and electrically connects the outer portion of the plurality of second conductive yarns 5151 to the body of the user. According to such arrangement, the electrostatic shielding for each of the electrode elements is implemented.

The interweaving manners of the plurality of second conductive yarns 5131, the plurality of second conductive yarns 5141, and the plurality of second conductive yarns 5151 may refer to the interweaving manner of the plurality of second conductive yarns 5111.

In some embodiments of the present disclosure, the conductive yarns (i.e., the second conductive yarns) are interwoven through the inner surface and the outer surface of the strap, such that the conductive yarns may form the conductive region on the outer surface to disperse static electricity and introduce the static electricity into the human body, thereby implementing the electrostatic shielding of the electrodes.

In some embodiments, as shown in FIGs. 5A-5P, the device 500 for monitoring a physiological signal may further be provided with a connection port 541 and a connection port 542 on the outer surface of the strap 510, and the fifth electrode element 521 and the sixth electrode element 522 may be electrically connected (conductively connected) to the connection port 541 and the connection port 542, respectively. The connection port 541 and the connection port 542 may be connected to a transmitter (not shown in the figures) (e.g., engaged in a male-female snap manner), and the transmitter may transmit the electrical signals (e.g., electrocardiogram potentials) collected by the fifth electrode element 521 and the sixth electrode element 522 to the processing device. The processing device may determine the physiological signal (e.g., the electrocardiogram signal) and/or physiological data (e.g., an electrocardiogram) of a monitored target (e.g., the monitored target 140) based on the electrical signals. The connection port 541 and the connection port 542 may also be directly connected to the processing device (not shown in the figures) (e.g., detachably connected by the magnetic attachment), and the processing device may directly obtain the electrical signals collected by the fifth electrode element 521 and the sixth electrode element 522 through the connection port 541 and the connection port 542, and determine the physiological signal (e.g., the electrocardiogram signal) and/or the physiological data (e.g., the electrocardiogram) of the monitored target (e.g., the monitored target 140) based on the electrical signals.

In some embodiments, the waterproof layer divides the strap into a first sub-strap and a second sub-strap that are mutually isolated, one of the two electrode elements is located on the first sub-strap, and the other of the two electrode elements is located on the second sub-strap.

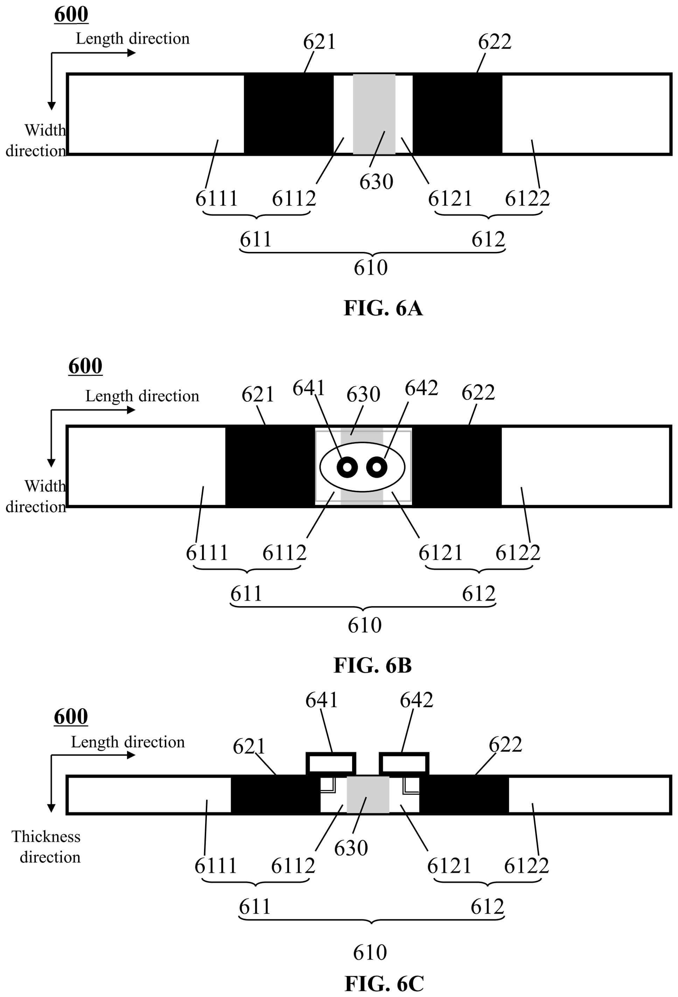

FIG. 6A is a structural diagram illustrating an inner surface of yet another device for monitoring a physiological signal according to some embodiments of the present disclosure; FIG. 6B is a structural diagram illustrating an outer surface of yet another device for monitoring a physiological signal according to some embodiments of the present disclosure; and FIG. 6C is a front view of yet another device for monitoring a physiological signal according to some embodiments of the present disclosure.

As shown in FIGs. 6A to 6C, a waterproof layer 630 divides a strap 610 into a first sub-strap 611 and a second sub-strap 612 that are mutually isolated, a seventh electrode element 621 is located on the first sub-strap 611, and an eighth electrode element 622 is located on the second sub-strap 612. The seventh electrode element 621 divides the first sub-strap 611 into a first sub-strap portion 6111 and a second sub-strap portion 6112, and the eighth electrode element 622 divides the second sub-strap 612 into a third sub-strap portion 6121 and a fourth sub-strap portion 6122.

In some embodiments, to obtain the device 600 for monitoring a physiological signal as shown in FIGs. 6A to 6C, a weaving process of the device 600 for monitoring a physiological signal may include: mixing and weaving the elastic yarn and the insulating yarn to form the first sub-strap portion 6111 and the second sub-strap portion 6112 of the first sub-strap 611 and the third sub-strap portion 6121 and the fourth sub-strap portion 6122 of the second sub-strap 612; weaving the waterproof insulating yarn to form the waterproof layer 630; weaving the first conductive yarn to form the seventh electrode element 621 and the eighth electrode element 622, and splicing the first sub-strap portion 6111, the seventh electrode element 621, the second sub-strap portion 6112, the waterproof layer 630, the third sub-strap portion 6121, the eighth electrode element 622, and the fourth sub-strap portion 6122 in sequence according to the positional relationship shown in FIGs. 6A to 6C (i.e., sequentially arranged in the length direction of the device 600 for monitoring a physiological signal are the first sub-strap portion 6111, the seventh electrode element 621, the second sub-strap portion 6112, the waterproof layer 630, the third sub-strap portion 6121, the eighth electrode element 622, and the fourth sub-strap portion 6122), thereby obtaining the device 600 for monitoring a physiological signal. The splicing method described above may be as follows: the adjacent side edges of the above portions (including the adjacent side edges between the first sub-strap portion 6111 and the seventh electrode element 621, between the seventh electrode element 621 and the second sub-strap portion 6112, between the second sub-strap portion 6112 and the waterproof layer 630, between the waterproof layer 630 and the third sub-strap portion 6121, between the third sub-strap portion 6121 and the eighth electrode element 622, and between the eighth electrode element 622 and the fourth sub-strap portion 6122) are sewn and spliced using yarns (e.g., the insulating yarn or the waterproof insulating yarn), or the adjacent side edges of the above portions are bonded and spliced using an adhesive (e.g., latex).

In some embodiments, to obtain the device 600 for monitoring a physiological signal as shown in FIGs. 6A to 6C, the strap 610, the waterproof layer 630, and the two electrode elements (including the seventh electrode element 621 and the eighth electrode element 622) included in the device 600 for monitoring a physiological signal may be integrally woven. The above integrated weaving process may be as follows: based on the yarns included in the strap 610 (including the first sub-strap 611 and the second sub-strap 612, where the first sub-strap 611 includes the first sub-strap portion 6111 and the second sub-strap portion 6112, and the second sub-strap 612 includes the third sub-strap portion 6121 and the fourth sub-strap portion 6122), the waterproof layer 630, and the electrode elements, where the strap 610 includes a mixed yarn of the elastic yarn and the insulating yarn, the waterproof layer 630 includes the waterproof insulating yarn, and the electrode elements include the first conductive yarn, the strap 610, the waterproof layer 630, and the two electrode elements are continuously woven according to the positional relationship shown in FIGs. 6A to 6C to obtain the formed physiological signal monitoring device 600. During the weaving process, when transitioning among the first sub-strap portion 6111, the seventh electrode element 621, the second sub-strap portion 6112, the waterproof layer 630, the third sub-strap portion 6121, the eighth electrode element 622, and the fourth sub-strap portion 6122, a transition weaving technique (e.g., tuck knitting) needs to be adopted.

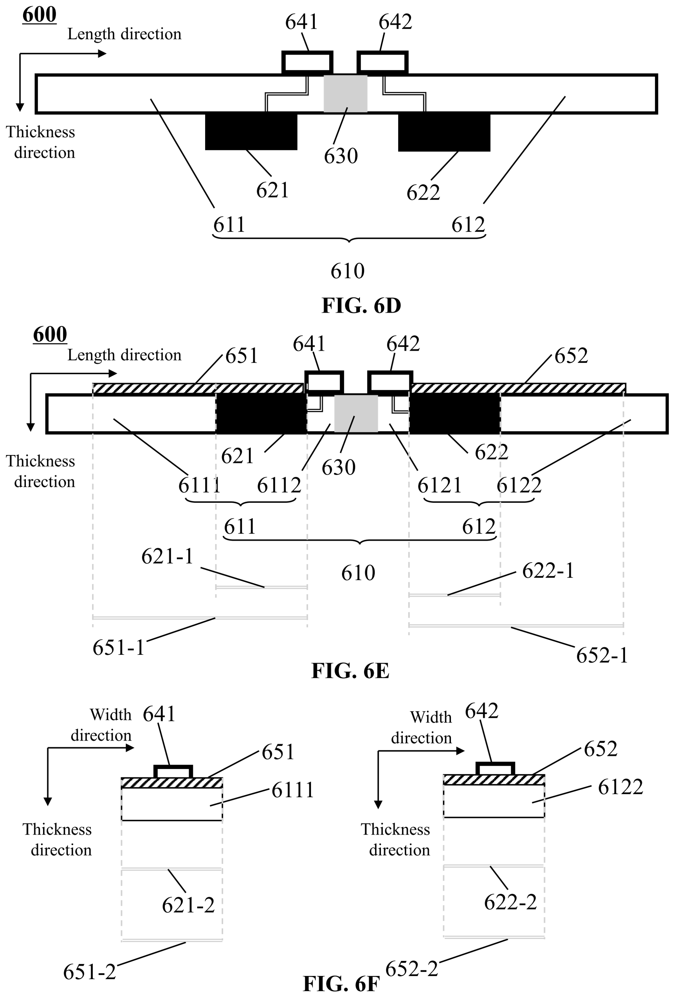

FIG. 6D is another front view of yet another device for monitoring a physiological signal according to some embodiments of the present disclosure.

As shown in FIG. 6D, the waterproof layer 630 divides the strap 610 into the first sub-strap 611 and the second sub-strap 612 that are mutually isolated, the seventh electrode element 621 is located on the inner surface of the first sub-strap 611, and the eighth electrode element 622 is located on the inner surface of the second sub-strap 612.

In some embodiments, to obtain the device 600 for monitoring a physiological signal as shown in FIG. 6D, a weaving process of the device 600 for monitoring a physiological signal may include: mixing and weaving the elastic yarn and the insulating yarn to form the first sub-strap 611 and the second sub-strap 612; weaving the waterproof insulating yarn to form the waterproof layer 630; weaving the first conductive yarn to form the seventh electrode element 621 and the eighth electrode element 622, splicing the first sub-strap 611, the waterproof layer 630, and the second sub-strap 612 in sequence according to the positional relationship shown in FIG. 6D (i.e., sequentially arranged in the length direction of the strap 610 are the first sub-strap 611, the waterproof layer 630, and the second sub-strap 612), and then splicing the seventh electrode element 621 and the eighth electrode element 622 respectively to the inner surfaces of the first sub-strap 611 and the second sub-strap 612, thereby obtaining the device 600 for monitoring a physiological signal. The splicing method described above may be as follows: the adjacent side edges of the above portions (including the adjacent side edges between the first sub-strap 611 and the waterproof layer 630, between the waterproof layer 630 and the second sub-strap 612, between the first sub-strap 611 and the seventh electrode element 621, and between the second sub-strap 612 and the eighth electrode element 622) are sewn and spliced using yarns (e.g., the insulating yarn or the waterproof insulating yarn), or the adjacent side edges of the above portions are bonded and spliced using an adhesive (e.g., latex).

In some embodiments, to obtain the device 600 for monitoring a physiological signal as shown in FIG. 6D, the strap 610, the waterproof layer 630, and two electrode elements (including the seventh electrode element 621 and the eighth electrode element 622) included in the device 600 for monitoring a physiological signal may be integrally woven. The above integrated weaving process may be as follows: based on the yarns included in the strap 610 (including the first sub-strap 611 and the second sub-strap 612), the waterproof layer 630, and the electrode elements, where the strap 610 includes a mixed yarn of the elastic yarn and the insulating yarn, the waterproof layer 630 includes the waterproof insulating yarn, and the electrode elements include the first conductive yarn, the strap 610, the waterproof layer 630, and the two electrode elements are continuously woven according to the positional relationship shown in FIG. 6D to obtain the formed device 600 for monitoring a physiological signal. During the weaving process, when transitioning among the first sub-strap 611, the second sub-strap 612, the waterproof layer 630, the seventh electrode element 621, and the eighth electrode element 622, a transition weaving technique (e.g., tuck knitting) needs to be adopted.

In some embodiments of the present disclosure, based on the device for monitoring a physiological signal as shown in FIGs. 6A to 6C, the waterproof layer may also prevent the two electrode elements from abnormal conduction due to water. Meanwhile, by reducing the size of the waterproof layer, the elasticity of the strap can be improved, thereby enhancing the wearing comfort.

In some embodiments, a first anti-static electrode is arranged on the first sub-strap, a second anti-static electrode is arranged on the second sub-strap, and the first anti-static electrode and the second anti-static electrode cover the first conductive yarn.

FIG. 6E is still another front view of yet another device for monitoring a physiological signal according to some embodiments of the present disclosure; and FIG. 6F is a schematic diagram illustrating a side projection of yet another device for monitoring a physiological signal according to some embodiments of the present disclosure. The left view and the right view in FIG. 6F are schematic side projection diagrams respectively observed from two opposite sides of the device 600 for monitoring a physiological signal.

As shown in FIG. 6E, a first anti-static electrode 651 is arranged on the first sub-strap 611, and a second anti-static electrode 652 is arranged on the second sub-strap 612, where the first anti-static electrode 651 covers the seventh electrode element 621, and the second anti-static electrode 652 covers the eighth electrode element 622, thereby enabling the first anti-static electrode 651 and the second anti-static electrode 652 to cover the first conductive yarn (the first conductive yarn refers to a yarn used for weaving the electrodes in the seventh electrode element 621 and the eighth electrode element 622).

The above-mentioned "covering" refers to that a projection of the first anti-static electrode 651 in the thickness direction of the strap 610 covers, in the length direction (and the width direction), a projection of the first conductive yarn in the seventh electrode element 621 in the thickness direction of the strap 610, and a projection of the second anti-static electrode 652 in the thickness direction of the strap 610 covers, in the length direction (and the width direction), a projection of the first conductive yarn in the eighth electrode element 622 in the thickness direction of the strap 610.

As shown in FIG. 6E, a projected long side 621-1 of the seventh electrode element 621 (i.e., a side of a projection region of the seventh electrode element 621 which is parallel to the length direction of the strap 610) is contained within a projected long side 651-1 of the first anti-static electrode 651 (i.e., a side of a projection region of the first anti-static electrode 651 which is parallel to the length direction of the strap 610), and a projected long side 622-1 of the eighth electrode element 622 (i.e., a side of a projection region of the eighth electrode element 622 which is parallel to the length direction of the strap 610) is contained within a projected long side 652-1 of the second anti-static electrode 652 (i.e., a side of a projection region of the second anti-static electrode 652 which is parallel to the length direction of the strap 610), such that the first anti-static electrode 651 covers the seventh electrode element 621 in the length direction of the strap 610, and the second anti-static electrode 652 covers the eighth electrode element 622 in the length direction of the strap 610.

As shown in FIG. 6F, a projected wide side 621-2 of the seventh electrode element 621 (i.e., a side of the projection region of the seventh electrode element 621 which is parallel to the width direction of the strap 610) is contained within a projected wide side 651-2 of the first anti-static electrode 651 (i.e., a side of the projection region of the first anti-static electrode 651 which is parallel to the width direction of the strap 610). A projected wide side 622-2 of the eighth electrode element 622 (i.e., a side of the projection region of the eighth electrode element 622 which is parallel to the width direction of the strap 610) is contained within a projected wide side 652-2 of the second anti-static electrode 652 (i.e., a side of the projection region of the second anti-static electrode 652 which is parallel to the width direction of the strap 610), such that the first anti-static electrode 651 covers the seventh electrode element 621 in the width direction of the strap 610, and the second anti-static electrode 652 covers the eighth electrode element 622 in the width direction of the strap 610.

In some embodiments, the first anti-static electrode 651 and the second anti-static electrode 652 are not electrically connected, so as to prevent the seventh electrode element 621 and the eighth electrode element 622 from being electrically connected through the first anti-static electrode 651 and the second anti-static electrode 652, thereby ensuring that the physiological signal collected has a higher signal-to-noise ratio (i.e., SNR).

In some embodiments of the present disclosure, the anti-static electrode may form a conductive region on the outer surface of the strap to disperse static electricity and introduce the static electricity into the human body, thereby implementing the electrostatic shielding of the electrodes.

In some embodiments, as shown in FIGs. 6A-6F, the device 600 for monitoring a physiological signal may further be provided with a connection port 641 and a connection port 642 on the outer surface of the strap 610, and the seventh electrode element 621 and the eighth electrode element 622 may be electrically connected (conductively connected) to the connection port 641 and the connection port 642, respectively. The connection port 641 and the connection port 642 may be connected to a transmitter (not shown in the figures) (e.g., connected in a male-female snap manner). The transmitter may transmit electrical signals (e.g., the electrocardiogram signal) collected by the seventh electrode element 621 and the eighth electrode element 622 to the processing device, and the processing device may determine a physiological signal (e.g., the electrocardiogram signal) and/or physiological data (e.g., an electrocardiogram) of a monitored target (e.g., the monitored target 140) based on the electrical signals. The connection port 641 and the connection port 642 may also be directly connected to the processing device (not shown in the figures) (e.g., detachably connected by magnetic attachment). The processing device may directly obtain the electrical signals collected by the seventh electrode element 621 and the eighth electrode element 622 through the connection port 641 and the connection port 642, and determine the physiological signal (e.g., the electrocardiogram signal) and/or physiological data (e.g., the electrocardiogram) of the monitored target (e.g., the monitored target 140) based on the electrical signals.

In some embodiments of the present disclosure, by providing the waterproof layer, the electrodes are connected to the strap through the waterproof layer. When the strap is exposed to liquid (e.g., sweat, water, or the like), the waterproof layer may prevent the liquid from spreading to the electrodes, thereby achieving waterproof protection of the electrodes. In addition, in the present disclosure, the strap, the waterproof layer, and/or the electrode elements are manufactured by integrated weaving or spliced weaving. On one hand, the manufacturing process of the device for monitoring a physiological signal can be simplified, and the process duration can be shortened, thereby facilitating mass production of the device for monitoring a physiological signal. On the other hand, the consistency of fabricating the device for monitoring a physiological signal can be improved, thereby enhancing the reliability of the device for monitoring a physiological signal and the quality of the physiological signals collected. Furthermore, the integrated weaving manner is advantageous for reducing the overall size of the device for monitoring a physiological signal and improving the comfort when the electrodes and the waterproof layer contact the human skin.

The basic concepts have been described above. Obviously, to a person skilled in the art, the above detailed description is merely an example and does not constitute a limitation to the present disclosure. Although not explicitly stated here, those skilled in the art may make various modifications, improvements and amendments to the present disclosure. These alterations, improvements, and modifications are intended to be suggested by the present disclosure, and are within the spirit and scope of the exemplary embodiments of the present disclosure.

## Claims

1. A device for monitoring a physiological signal, comprising:
a strap, configured to be worn on a body of a user;
two electrode elements, arranged at intervals in a length direction of the strap, each of the two electrode elements including an electrode configured to contact the body of the user to collect the physiological signal; and
a waterproof layer, wherein the two electrode elements are both connected to the strap through the waterproof layer, the strap includes at least an elastic yarn and an insulating yarn, the waterproof layer includes a waterproof insulating yarn, and the strap and the waterproof layer are formed by integrated weaving.

2. The device of claim 1, wherein the two electrode elements are located at two sides of a median sagittal plane of a human body, and the two electrode elements are configured to collect electrocardiogram signals of the human body.

3. The device of claim 1, wherein the electrode includes a first conductive yarn, and the electrode, the waterproof layer, and the strap are formed by integrated weaving.

4. The device of claim 3, wherein each of the two electrode elements includes an electrode support layer, the electrode support layer is located between the electrode and the waterproof layer, the electrode support layer at least includes a yarn different from the first conductive yarn, and the electrode support layer and the electrode are formed by integrated weaving.

5. The device of claim 4, wherein in a non-wearing state, an inner surface of each of the two electrode elements protrudes beyond an inner surface of the strap, and a protrusion distance is in a range of 0.1 mm to 5 mm.

6. The device of claim 3, wherein the waterproof layer is woven on an inner surface of the strap, and the two electrode elements are woven on a side of the waterproof layer facing away from the strap.

7. The device of claim 6, wherein the strap includes a first region and a second region, an elasticity of the first region is less than an elasticity of the second region, the first region is a projected region of the electrode on the strap, and the second region does not overlap with the first region.

8. The device of claim 7, wherein the first region does not include the elastic yarn.

9. The device of claim 6, wherein the strap includes a second conductive yarn, and the second conductive yarn implements electrostatic shielding for the two electrode elements.

10. The device of claim 9, wherein a first portion of the second conductive yarn is located on an outer surface of the strap or between the inner surface and the outer surface of the strap, such that each of the two electrode elements is at least partially located between the first portion of the second conductive yarn and the body of the user, and a second portion of the second conductive yarn extends to the inner surface of the strap and electrically connects the first portion of the second conductive yarn to the body of the user to implement the electrostatic shielding for each of the two electrode elements.

11. The device of claim 10, wherein the second conductive yarn is interwoven through the inner surface and the outer surface of the strap.

12. The device of claim 10, wherein the strap is at least formed by mixed weaving of the elastic yarn, the insulating yarn, and the second conductive yarn.

13. The device of claim 10, wherein each of the first portion of the second conductive yarn and the second portion of the second conductive yarn includes a plurality of conductive channels arranged in parallel in a width direction of the strap, and each conductive channel of the plurality of conductive channels is formed by weaving the second conductive yarn in a wave shape in the length direction of the strap.

14. The device of claim 10, wherein the two electrode elements include a first electrode element and a second electrode element that are arranged at intervals in the length direction of the strap, the first portion of the second conductive yarn forms one or more conductive regions, and the one or more conductive regions cover the first electrode element and the second electrode element.

15. The device of claim 3, wherein the waterproof layer and the strap are woven in a spliced manner, and the waterproof layer extends through an inner surface and an outer surface of the strap in a thickness direction of the strap.

16. The device of claim 15, wherein the two electrode elements are woven on an inner side of the waterproof layer.

17. The device of claim 15, wherein the electrode and the waterproof layer are woven in a spliced manner, and the waterproof layer is located between the strap and the two electrode elements.

18. The device of claim 17, wherein the strap includes a second conductive yarn, the second conductive yarn is interwoven through the inner surface and the outer surface of the strap, and the second conductive yarn implements electrostatic shielding for each of the two electrode elements.

19. The device of claim 3, wherein the waterproof layer divides the strap into a first sub-strap and a second sub-strap that are mutually isolated, one of the two electrode elements is located on the first sub-strap, and the other of the two electrode elements is located on the second sub-strap.

20. The device of claim 19, wherein a first anti-static electrode is arranged on the first sub-strap, a second anti-static electrode is arranged on the second sub-strap, and the first anti-static electrode and the second anti-static electrode cover the first conductive yarn.

21. The device of claim 1, wherein two connection ports are provided on the strap, the two connection ports are electrically connected to the two electrode elements, respectively, to implement data transmission between the two electrode elements and a processing device, and the processing device is detachably connected to the two connection ports by magnetic attachment.

22. The device of claim 21, wherein each of the two connection ports is spaced apart from yarns on the strap, and a waterproof insulating material is filled between the two connection ports and the strap.

23. The device of claim 21, wherein each of the two electrode elements is connected to a corresponding connection port via a conductive yarn wrapped with an insulating material.
